(19) Europäisches Patentamt
European Patent Office
Office européen des brevets

(11) EP 4 368 197 A1

(12) EUROPEAN PATENT APPLICATION
published in accordance with Art. 153(4) EPC

(43) Date of publication:
**15.05.2024 Bulletin 2024/20**

(21) Application number: **22837720.6**

(22) Date of filing: **06.07.2022**

(51) International Patent Classification (IPC):
***A61K 38/43*** *(2006.01)* ***A61K 31/7088*** *(2006.01)*
***A61K 48/00*** *(2006.01)* ***A61P 21/00*** *(2006.01)*
***A61P 25/00*** *(2006.01)* ***A61P 43/00*** *(2006.01)*
***C07H 19/067*** *(2006.01)* ***C12N 9/99*** *(2006.01)*
***C12N 15/52*** *(2006.01)* ***C12N 15/85*** *(2006.01)*
***C12Q 1/68*** *(2018.01)* ***G01N 33/15*** *(2006.01)*

(52) Cooperative Patent Classification (CPC):
**A61K 31/7088; A61K 38/43; A61K 48/00; A61P 21/00; A61P 25/00; A61P 43/00; C07H 19/067; C12N 9/99; C12N 15/52; C12N 15/85; C12Q 1/68; G01N 33/15**

(86) International application number:
**PCT/JP2022/026887**

(87) International publication number:
**WO 2023/282306 (12.01.2023 Gazette 2023/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.07.2021 JP 2021111955**

(71) Applicant: **The University of Tokyo
Bunkyo-ku, Tokyo 113-8654 (JP)**

(72) Inventors:
- **SUZUKI, Tsutomu
Tokyo 113-8654 (JP)**
- **TOMODA, Ena
Tokyo 113-8654 (JP)**
- **SUZUKI, Takeo
Tokyo 113-8654 (JP)**
- **NAGAO, Asuteka
Tokyo 113-8654 (JP)**

(74) Representative: **MacLean, Martin Robert et al
Mathys & Squire
The Shard
32 London Bridge Street
London SE1 9SG (GB)**

(54) **PHARMACEUTICAL COMPOSITION FOR TREATING OR PREVENTING DISEASE ASSOCIATED WITH REDUCTION OR DEFECT OF tM5U MODIFICATION**

(57) The present invention provides a pharmaceutical composition for the treatment or prevention of a disease(s) caused by reduction or deficiency in 5-taurinomethyluridine ($\tau m^5U$) modification of mitochondrial tRNA, the composition comprising mitochondrial tRNA translation optimization 1 (MT01) or nucleic acid encoding MT01, wherein MT01 is administered to a patient in an excess amount, or MT01 is overexpressed in a patient to whom the nucleic acid has been administered.

Fig. 4

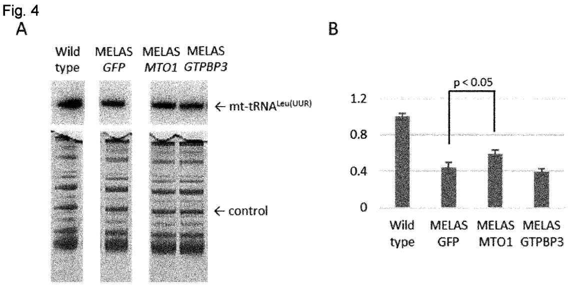


EP 4 368 197 A1

## Description

### Technical Field

The present invention broadly relates, inter alia, to a pharmaceutical composition for treating or preventing a disease(s) caused by reduction or deficiency in 5-taurinomethyluridine ($\tau m^5U$) modification of mitochondrial tRNA, and to novel applications of water-soluble carbodiimides.

### Background Art

Mitochondrial encephalomyopathy (mitochondrial encephalomyopathy, lactic acidosis, and stroke-like episodes) (MELAS) is one of the three major types of mitochondrial diseases and characteristically presents stroke-like symptoms. Approximately 80% of MELAS patients have an A3243G mutation in the mitochondrial genome and approximately 10% of patients have a T3271 C mutation. These mutations are present in the mitochondrial $tRNA^{Leu(UUR)}$ gene.

These mutant tRNAs actually have a reduced half-life and a reduced steady-state level in the cell. In addition, it has been found that for the mutant mitochondrial $tRNA^{Leu(UUR)}$, the 5-taurinomethyluridine ($\tau m^5U$) modification of the first position of the anticodon is substantially reduced, and that, of the two corresponding codons UUA and UUG, the ability to decode the UUG codon is reduced (Non-Patent Documents 1 and 2). This is a primary factor in reduction in the activity of the mitochondrial respiratory chain complex I.

It is thought, as a consequence, that mutations in the mitochondrial genome in MELAS patients impair recognition by $\tau m^5U$-modifying enzymes (complex of MTO1 and GTPBP3).

Citation List

Non-Patent Documents

Non-Patent Document 1: Nature Reviews Molecular Cell Biology 22(6), 375-392 (2021)

Non-Patent Document 2: Journal of Japanese Biochemical Society 92(3): 431-446 (2020)

Non-Patent Document 3: PNAS. 102, 7127-7132 (2005)


### Summary

Technical Problem

It has been predicted that MELAS mutant tRNA becomes structurally unstable because the mutation site is an important site for higher order structure formation in the tRNA. Due to this, it is necessary, in order to treat or prevent a disease(s) caused by reduction or deficiency in $\tau m^5U$ modification, to stabilize the structure of the mutant tRNA and restore the $\tau$m5U modification.

A primer extension technique has previously been used to detect $\tau m^5U$ modification. In this primer extension technique, a reverse transcription reaction is carried out using a radiolabeled DNA primer that has a sequence complementary to the RNA and the modification rate is measured utilizing the inhibition of cDNA extension due to RNA modification. It is highly sensitive and requires less than 1 $\mu$g of total RNA, making it possible to carry out an analysis with rare specimens and clinical samples.

In previous research in a laboratory with which an inventor is affiliated, $\tau m^5U$ modification was successfully detected using the primer extension technique (PE technique) and using a reverse transcriptase from Maloney murine leukemia virus (Non-Patent Document 3). However, because this technique was successful only with a specific reverse transcriptase and required that stringent reaction conditions be determined, the development of a more convenient practical technique has been desired.

Solution to Problem

The present inventors have discovered that $\tau m^5U$ modification is recovered by an overexpression of MTO1, which constitutes a $\tau m^5U$-modifying enzyme, in MELAS cells, and that the $\tau m^5U$ modification is conveniently detected by a reverse transcription reaction of mitochondrial $tRNA^{Leu(UUR)}$ in the presence of a water-soluble carbodiimide.

Thus, the present invention encompasses the following inventions.

[1] A pharmaceutical composition for the treatment or prevention of a disease(s) caused by reduction or deficiency in 5-taurinomethyluridine ($\tau m^5U$) modification of mitochondrial tRNA, the composition comprising mitochondrial tRNA translation optimization 1 (MTO1) or nucleic acid encoding MTO1, wherein MTO1 is administered to a patient in an excess amount, or MTO1 is overexpressed in a patient to whom the nucleic acid has been administered.

[2] The pharmaceutical composition according to [1], wherein a $\tau m^5U$ modification rate is increased by the administration of MTO1 or the nucleic acid, in comparison to that prior to the administration.

[3] The pharmaceutical composition according to [2], wherein a $\tau m^5U$ modification rate is increased in comparison to the administration of excess GTP-binding protein 3 (GTPBP3) or in comparison to the overexpression of nucleic acid encoding GTPBP3.

[4] The pharmaceutical composition according to any of [1] to [3], wherein the nucleic acid encoding MTO1 has:

(i) nucleic acid consisting of a base sequence of SEQ ID NO: 1;

(ii) nucleic acid capable of hybridizing under stringent conditions with nucleic acid consisting of a base sequence complementary to the base sequence of SEQ ID NO: 1, and encoding protein having a $\tau m^5U$ modification activity;

(iii) nucleic acid consisting of a base sequence provided by deletion, substitution, or addition of one or a few bases in the base sequence of SEQ ID NO: 1, and encoding protein having a $\tau m^5U$ modification activity;

(iv) nucleic acid consisting of a base sequence having an at least 80% sequence identity with SEQ ID NO: 1, and encoding protein having a $\tau m^5U$ modification activity;

(v) nucleic acid consisting of a base sequence encoding protein consisting of the amino acid sequence of SEQ ID NO: 2;

(vi) nucleic acid capable of hybridizing under stringent conditions with nucleic acid consisting of a base sequence encoding protein consisting of the amino acid sequence of SEQ ID NO: 2, and encoding protein having a $\tau m^5U$ modification activity;

(vii) nucleic acid consisting of a base sequence encoding protein consisting of an amino acid sequence provided by deletion, substitution, or addition of one or a few amino acids in the amino acid sequence of SEQ ID NO: 2, with the protein having a $\tau m^5U$ modification activity; or

(viii) nucleic acid consisting of a base sequence encoding protein consisting of an amino acid sequence having a sequence identity of at least 80% with SEQ ID NO: 2, with the protein having a $\tau m^5U$ modification activity.

[5] The pharmaceutical composition according to any of [1] to [4], wherein the nucleic acid encoding MTO1 is contained in a vector.

[6] The pharmaceutical composition according to [5], wherein the vector is a viral vector or a plasmid.

[7] The pharmaceutical composition according to [6], wherein the viral vector is selected from the group consisting of retrovirus vectors, lentivirus vectors, adenovirus vectors, and adeno-associated virus vectors.

[8] The pharmaceutical composition according to any of [1] to [7], wherein the MTO1 or nucleic acid encoding MTO1 is administered systemically.

[9] The pharmaceutical composition according to any of [1] to [8], wherein the disease(s) caused by reduction or deficiency in $\tau m^5U$ modification is mitochondrial encephalomyopathy.

[10] A vector comprising nucleic acid encoding MTO1.

[11] A method of detecting $\tau m^5U$ modification of mitochondrial tRNA, the method comprising: a step of performing,

in the presence of a water-soluble carbodiimide, a reverse transcription reaction on mitochondrial $tRNA^{Leu(UUR)}$ contained in a sample derived from a subject, wherein

the presence of $\tau m^5U$ modification is indicated when the reverse transcription reaction stops earlier than a $\tau m^5U$ at position 34 of the mitochondrial $tRNA^{Leu(UUR)}$ or stops at a position corresponding to position 33, or

the absence of $\tau m^5U$ modification is indicated when the reverse transcription reaction progresses to or beyond a position corresponding to position 32.

[12] The method according to [11], wherein, when $\tau m^5U$ modification is present, the reverse transcription reaction stops at a position of adenosine at position 35 or a position of uridine at position 33.

[13] The method according to [11] or [12], wherein the reverse transcription reaction is a primer extension reaction using a reverse transcriptase.

[14] The method according to any of [11] to [13], wherein the method further comprises a step of measuring a $\tau m^5U$ modification rate.

[15] The method according to any of [11] to [14], wherein the mitochondrial $tRNA^{Leu(UUR)}$ has an A3243G mutation.

[16] The method according to any of [11] to [15], wherein the water-soluble carbodiimide is selected from the group consisting of N-cyclohexyl-N'-β-(4-methylmorpholinium) ethylcarbodiimide (CMC), 1-ethyl-3-(3-dimethylaminopropyl)carbodiimide (EDC), 4-(4,6-dimethoxy-1,3,5-triazin-2-yl)-4-methylmorpholinium chloride (DMT-MM), and salts thereof.

[17] A method of screening for drugs that treat or prevent a disease(s) caused by reduction or deficiency in $\tau m^5U$ modification, the method comprising: a step of performing, in the presence of a water-soluble carbodiimide, a reverse transcription reaction on mitochondrial $tRNA^{Leu(UUR)}$ contained in a sample derived from a subject that has been treated with a candidate substance; and a step of measuring a $\tau m^5U$ modification rate, wherein, when the $\tau m^5U$ modification rate is increased in comparison to that for a sample where treatment with the candidate substance has not been carried out, the candidate substance is selected as a drug that treats or prevents a disease(s) caused by reduction or deficiency in $\tau m^5U$ modification.

[18] A method for in vitro detection of a disease(s) caused by reduction or deficiency in $\tau m^5U$ modification of mitochondrial tRNA, the method comprising: a step of performing, in the presence of a water-soluble carbodiimide, a reverse transcription reaction on mitochondrial $tRNA^{Leu(UUR)}$ contained in a sample derived from a subject, wherein

it is indicated that the subject is not suffering from a disease(s) caused by reduction or deficiency in $\tau m^5U$ modification when the reverse transcription reaction stops earlier than a $\tau m^5U$ at position 34 of the mitochondrial $tRNA^{Leu(UUR)}$ or stops at a position corresponding to position 33, or

it is indicated that the subject is suffering from a disease(s) caused by reduction or deficiency in $\tau m^5U$ modification when the reverse transcription reaction progresses to or beyond a position corresponding to position 32 in the mitochondrial $tRNA^{Leu(UUR)}$ .

[19] A method for determining whether or not a subject that has or is suspected of having a disease(s) caused by reduction or deficiency in $\tau m^5U$ modification is to be a target for treatment with taurine, the method comprising: a step of performing, in the presence of a water-soluble carbodiimide, a reverse transcription reaction on mitochondrial $tRNA^{Leu(UUR)}$ contained in a sample derived from a subject, wherein

it is indicated that the subject can be a target for a treatment using taurine, when the reverse transcription reaction stops earlier than a $\tau m^5U$ at position 34 of the mitochondrial $tRNA^{Leu(UUR)}$ or stops at a position corresponding to position 33, or

it is indicated that the subject can be a target for a treatment using taurine when the reverse transcription reaction progresses to or beyond a position corresponding to position 32 in the mitochondrial $tRNA^{Leu(UUR)}$.

[20] A compound having a structure below or a salt thereof:

[C1]

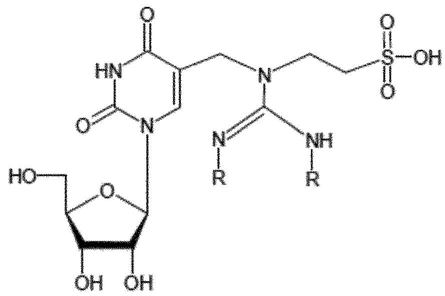

wherein R is a saturated or unsaturated hydrocarbon group, which may have a substituent, and two R's may be the same as each other.

[21] An inhibitor of a reverse transcription reaction on τ$m^5$U modification-comprising nucleic acid, the inhibitor comprising a water-soluble carbodiimide as an active ingredient.

[22] A kit comprising a reverse transcriptase and the reverse transcription reaction inhibitor according to [21].

Advantageous Effects of Invention

In patients suffering from a disease(s) caused by reduction or deficiency in τ$m^5$U modification, the τ$m^5$U modification rate can be increased by the overexpression of MTO1 and mitochondrial function can then be stimulated.

In addition, τ$m^5$U modification can be conveniently detected by performing, in the presence of a water-soluble carbodiimide, a reverse transcription reaction on mitochondrial $tRNA^{Leu(UUR)}$.

## Brief Description of Drawings

Fig. 1 relates to construction of a stable MTO1-expressing strain: A) MTO1_FLAG construct; B) immunostaining of MTO1 in the stable MTO1-expressing strain.

Fig. 2 relates to RT-qPCR results for MTO1- and GTPBP3-overexpressing strains.

Fig. 3 relates to examination of taurine modification using a primer extension technique and a water-soluble carbodiimide: A) τ$m^5$U derivatized with CMC; B) quantitative study of τ$m^5$U modification rate; C) calibration curve for τ$m^5$U modification rate; and D, E) measurement of τ$m^5$U modification rate in stable overexpressing strains.

Fig. 4 relates to analysis of the steady-state level of mitochondrial $tRNA^{Leu(UUR)}$ by Northern blotting.

Fig. 5 relates to evaluation of ability to synthesize mitochondrial proteins using a pulse-labeling technique: A) left: visualization by image analyzer of [$^{35}$S]-labeled mitochondrial protein, right: gel after CBB staining; B) standardization of each quantified band with wild-type strain; C) quantitation of all bands within blue frame.

Fig. 6 relates to reverse transcription reaction on mitochondrial $tRNA^{Leu(UUR)}$.

Fig. 7 relates to inhibition of reverse transcription reaction on mitochondrial $tRNA^{Leu(UUR)}$ by water-soluble carbodiimide.

Fig. 8-1 shows SEQ ID NO: 1.

Fig. 8-2 is continuation of Fig. 8-1.

Fig. 9-1 shows SEQ ID NO: 2.

Fig. 9-2 is continuation of Fig. 9-1.

Fig. 9-3 is continuation of Fig. 9-2.

Fig. 9-4 is continuation of Fig. 9-3.

Fig. 9-5 is continuation of Fig. 9-4.

## Description of Embodiments

Embodiments of the present invention (referred to below as the "present embodiments") are described in the following, but the following embodiments should not be understood as limitations on the scope of the present invention.

(The pharmaceutical composition)

A first embodiment provides a pharmaceutical composition for treating or preventing a disease(s) caused by reduction or deficiency in the $\tau m^5U$ modification of mitochondrial tRNA, wherein the pharmaceutical composition comprises MTO1 or nucleic acid encoding MTO1, and with which MTO1 is administered in an excess amount or MTO1 is overexpressed in a patient to whom the nucleic acid has been administered.

The $\tau m^5U$ (5-taurinomethyluridine) modification is a modification of mitochondrial tRNA and is generally seen in a wide range of animals, e.g., vertebrates (e.g., humans, mice, bovines, felines) and protochordates (ascidians) to mollusks (squid). As used herein, the "$\tau m^5U$ modification" refers to the taurine modification of mitochondrial $tRNA^{Leu(UUR)}$, a pos-transcriptional modification in which a taurinomethyl group is attached to the 5-position of the uracil present at position 34 (the wobble position), corresponding to the first position of anticodon of mitochondrial $tRNA^{Leu(UUR)}$.

A deficiency or reduction in mitochondrial tRNA modification is frequently a cause of disease. A point mutation in a tRNA gene is regarded as a cause of MELAS, which is characterized by strokes, and MERRF (myoclonic epilepsy associated with ragged-red fibers), which is characterized by epilepsy, and in such mutant mitochondrial tRNAs, taurine modification is substantially reduced and the uridine remains unmodified.

Such reduction in taurine modification can be evaluated using the proportion of $\tau m^5U$ modification at position 34 (also referred to as the $\tau m^5U$ modification rate) that is present in a specific tRNA, i.e., mitochondrial $tRNA^{Leu(UUR)}$. As used herein, the "$\tau m^5U$ modification rate" can be given by the following formula.

$$\tau m^5U \text{ modification rate } (\%) = \tau m^5U/(\tau m^5U + U) \times 100$$

When a reverse transcription reaction is carried out on mitochondrial $tRNA^{Leu(UUR)}$, the reaction proceeds according to the course shown in Fig. 6, but in the case of $\tau m^5U$ modification the reverse transcription reaction stops at a position corresponding to position 33 of the mitochondria $tRNA^{Leu(UUR)}$ (Fig. 7, center). However, when the reverse transcription reaction is carried out on a template of mitochondrial $tRNA^{Leu(UUR)}$ in the presence of a water-soluble carbodiimide, e.g., CMC, the $\tau m^5U$ derivatized by a contact with the water-soluble carbodiimide causes the reverse transcription reaction to stop at a position corresponding to position 35 (Fig. 7, right). On the other hand, in the case of the unmodified U, the reverse transcription reaction proceeds and the extension reaction is ended by the incorporation of dideoxyguanosine triphosphate (ddGTP) at the C at position 32 (Fig. 7, left).

In the case of quantitation of the extension reaction product using, for example, denaturing polyacrylamide gel electrophoresis, the band intensities for position 32, position 33, and position 35 can be quantitated, and the band intensity ratio, as calculated using the following formula, can also be associated with the $\tau m^5U$ modification rate by constructing a calibration curve between the band intensity ratio and the $\tau m^5U$ modification rate calculated from the sample mixing ratio.

band intensity ratio = (band intensity for position 33 + band intensity for position 35)/(total value of band intensities for position 32, position 33, and position 35)

As used herein, a "reduction in $\tau m^5U$ modification" means reduction in the $\tau m^5U$ modification rate, in comparison to normal $tRNA^{Leu(UUR)}$ in which taurine modification is formed, to a degree that causes a disease such as MELAS or

MERRF. For example, the τ$m^5$U modification rate in myoblasts from certain MELAS patients is less than 20%. However, the τ$m^5$U modification rate varies depending on differences in the cells, tissues, and patients. For example, it is known that the τ$m^5$U modification rate in HeLa cells, which are commonly used cells derived from human cervical cancer, is 96.3%.

Mitochondrial encephalomyopathies, e.g., MELAS and MERRF, are examples of a disease(s) caused by reduction or deficiency in τ$m^5$U modification, but the present invention is not limited to these diseases.

In order to treat or prevent a disease(s) caused by reduction or deficiency in τ$m^5$U modification, MTO1, which is a taurine-modification enzyme, is administered in an excess amount to a patient, or MTO1 is overexpressed in a patient to whom MTO1-encoding nucleic acid has been administered.

As used herein, an "excess amount" of MTO1 and "overexpression" for MTO1-encoding nucleic acid mean a higher level in comparison to a control, for example, in comparison to the amount of MTO1 present in the body of a patient prior to the administration of the pharmaceutical composition, and means a condition that is at least approximately 1%, 5%, 25%, 50%, 75%, 100%, 125%, 150%, 175%, 200%, 250%, 300%, 350%, 400%, 450%, 500%, 600%, 700%, 800%, 900%, or at least 1000% higher than the control. 500% to at least 1000% is preferred. In a preferred embodiment, excess amount and overexpression mean a level at which, by the administration to a patient of MTO1 or MTO1-encoding nucleic acid, the τ$m^5$U modification rate is raised relative to prior to the administration. Thus, when MTO1 is administered directly, its excess amount is determined as appropriate in correspondence to the patient and the desired effect.

The overexpression of MTO1 can be achieved by methods well known to the person skilled in the art. For example, overexpression may be achieved by introducing an MTO1-encoding nucleic acid into the cells of a patient, for example, utilizing a lentivirus- or retrovirus-based gene transfer system, and inducing expression. When gene expression is performed using a viral gene transfer vector, the gene may be operably linked downstream from a suitable promoter, and this may be inserted into the gene transfer vector followed by transduction into cells and expression of the target gene.

As long as the desired function as MTO1 is exhibited, the MTO1 used for the administration of an excess amount of MTO1 may be a protein regarded as a fragment of MTO1 or as an MTO1 variant.

In the case of the administration of an MTO1-encoding nucleic acid to a patient for the overexpression of MTO1 in the body of the patient, the nucleic acid that is administered may be any nucleic acid capable of expressing protein having the desired function as MTO1. A vector comprising MTO1-encoding nucleic acid can be constructed, using well known methods, as a vector system that can express protein having the desired function as MTO1.

"Operably" linked indicates that, for example, the subject gene and a promoter are linked such that the desired expression, e.g., of an MTO1-encoding gene, is achieved by the promoter. A known promoter can be used. In addition, the promoter may be genetically modified to increase transcription of the MTO1-encoding gene.

When the MTO1-encoding nucleic acid is contained in a vector, the vector may be a viral vector or a plasmid. The viral vector can be selected from known vectors, for example, from the group consisting of retrovirus vectors, lentivirus vectors, adenovirus vectors, and adeno-associated virus vectors. The vector may be a self-replicating vector, for example, a plasmid, an extrachromosomal element, a minichromosome, or an artificial chromosome.

The MTO1-encoding nucleic acid may comprise the following, although this is not intended as a limitation:

(i) nucleic acid consisting of the base sequence consisting of SEQ ID NO: 1;

(ii) nucleic acid capable of hybridizing, under stringent conditions and preferably under highly stringent conditions, with nucleic acid consisting of a base sequence complementary to the base sequence of SEQ ID NO: 1, and encoding protein having a τ$m^5$U modification activity;

(iii) nucleic acid consisting of a base sequence provided by the deletion, substitution, or addition of one or a few bases, and preferably 1, 2, 3, 4, 5, 6, 7, 8, 9, or 10 per 100 bases, in the base sequence of SEQ ID NO: 1, and encoding protein having a τ$m^5$U modification activity;

(iv) nucleic acid consisting of a base sequence having a sequence identity of at least 80% and preferably at least 90%, at least 91%, at least 92%, at least 93%, at least 94%, at least 95%, at least 96%, at least 97%, at least 98%, or at least 99% with SEQ ID NO: 1, and encoding protein having a τ$m^5$U modification activity;

(v) nucleic acid consisting of a base sequence encoding protein consisting of the amino acid sequence of SEQ ID NO: 2;

(vi) nucleic acid encoding protein having a τ$m^5$U modification activity and capable of hybridizing under stringent conditions, and preferably under highly stringent conditions, with nucleic acid consisting of a base sequence encoding protein consisting of the amino acid sequence of SEQ ID NO: 2;

(vii) nucleic acid consisting of a base sequence encoding protein that comprises an amino acid sequence provided by the deletion, substitution, or addition of one or a few amino acids, and preferably 1, 2, 3, 4, 5, 6, 7, 8, 9, or 10 amino acids per 100 amino acids, in the amino acid sequence of SEQ ID NO: 2, wherein the protein has a $\tau m^5U$ modification activity; or

(viii) nucleic acid consisting of a base sequence encoding protein that comprises an amino acid sequence having a sequence identity of at least 80% and preferably at least 90%, at least 91%, at least 92%, at least 93%, at least 94%, at least 95%, at least 96%, at least 97%, at least 98%, or at least 99% with SEQ ID NO: 2, wherein the protein has a $\tau m^5U$ modification activity.

As used herein, "nucleic acid" means not only DNA, but also RNA, e.g., mRNA, or their salts.

Herein, stringent conditions refer to conditions under which a specific hybridazation is formed and nonspecific hybridazation are not formed. Stringent conditions can be readily determined by the person skilled in the art, and are generally empirical experimental conditions that depend on the base length of the nucleic acid, the washing temperature, and the salt concentration of the buffer. In general, the temperature for proper annealing is higher at a longer base length and is lower at a shorter base length. Hybridazation generally depends on the reannealing capability in an environment in which the complementary strands are somewhat below their melting temperature.

As used herein, "highly stringent conditions" refer to conditions designed to enable the hybridization of DNA strands having a high degree of complementarity in their nucleic acid sequence and to exclude the hybridization of DNA having a significantly large number of mismatches. Stringency can vary, for example, with the concentration of the sodium chloride/sodium citrate (SSC). For example, the manual for the ECL Direct Nucleic Acid Labeling and Detection System (Amersham Pharmacia Biotech) states that a highly stringent condition can be achieved by replacing the 0.1X SSC constituting the primary wash buffer with $0.50 \times$ SSC.

The $\tau m^5U$ modification rate is substantially increased when MTO1 is administered in an excess amount to a patient or when MTO1 is overexpressed in a patient to whom MTO1-encoding nucleic acid has been administered. It is thought that this increase in the $\tau m^5U$ modification rate exceeds the increase in the $\tau m^5U$ modification rate by the administration of GTPBP3, which constitutes a $\tau m^5U$-modifying enzyme together with MTO1, in excess or by the overexpression of a GTPBP3-encoding nucleic acid.

The dosage form, method of use, and dose of the MTO1 or MTO1-encoding nucleic acid can be determined based on general knowledge with regard to polymer drugs or nucleic acid drugs, respectively. For example, the dosage form may take the form of a parenteral formulation such as an injectable or an infusion. Carriers or vehicles that can be used in such parenteral formulations includes aqueous vehicles such as physiological saline and isotonic solutions.

The pharmaceutical composition may comprise ingredients such as pharmaceutically acceptable buffers, stabilizers, preservatives, and other additives. Pharmaceutically acceptable ingredients are well known to the person skilled in the art, and the person skilled in the art can, operating within the range of ordinary skill, select and use same according to the form of the formulation from ingredients listed in standards or norms, e.g., the Japanese Pharmacopoeia, 17th Edition.

The pharmaceutical composition can be administered orally or parenterally (locally, rectally, intravenous administration, intraarterial administration, intramuscularly, subcutaneously, and so forth). The method of administration is not particularly limited, but systemic administration by injection or infusion is preferred, for example, intravenous administration, intraarterial administration, and so forth. In another embodiment, administration to the periphery associated with the central nervous system (CNS) is also preferred. From the viewpoint of suppressing strokes in MELAS, the pharmaceutical composition is preferably introduced to the vascular endothelial cells of the brain. More preferably, it is administered to skeletal muscles systemically.

In another embodiment, there is provided a method for treating or preventing a disease(s) caused by reduction or deficiency in $\tau m^5U$ modification of mitochondrial tRNA, wherein the method comprises a step of administering MTO1 or MTO1-encoding nucleic acid to a subject wherein the MTO1 is administered in an excess amount or MTO1 is overexpressed in a patient to whom the nucleic acid has been administered.

Such a method may be carried out in combination with another therapeutic method used for the treatment or prevention of a disease(s) caused by reduction or deficiency in $\tau m^5U$ modification. For example, combination with the use of a pharmaceutical composition of, e.g., taurine, taurine chloramine, taurine precursor, tauroursodeoxycholic acid (TUDCA), and so forth, which are known as therapeutic agents for MELAS, may be considered. Among these, high-dose taurine supplementation therapy is preferred because its effectiveness in suppressing the recurrence of stroke-like attacks in MELAS has been confirmed. The MTO1 or MTO1-encoding nucleic acid may be administered simultaneously with another active ingredient, such as taurine, or may be administered at a different time. When administered simultaneously, the active ingredients may be included in the same pharmaceutical composition.

The subjects for the administration of MTO1 or MTO1-encoding nucleic acid are not limited to humans, but non-human vertebrates (e.g., monkeys, mice, rats, hamsters, guinea pigs, rabbits, cats, dogs, pigs, bovines, horses,

sheep, birds, reptiles, amphibians, fish), protochordates, mollusks, and so forth that can suffer from a disease(s) caused by reduction or deficiency in $\tau m^5U$ modification are also included in this scope.

Instead of administering MTO1 or MTO1-encoding nucleic acid, or in parallel with the administration of MTO1 or MTO1-encoding nucleic acid, a treatment that enhances the expression of MTO1 in the patient can also be carried out. For example, the enhanced expression of MTO1 can be induced by enhancing the subject's own endogenous MTO1 expression activity.

(The vector)

A vector comprising MTO1-encoding nucleic acid is provided in a second embodiment.

The MTO1-encoding nucleic acid may be as follows:

(i) nucleic acid consisting of the base sequence consisting of SEQ ID NO: 1;

(ii) nucleic acid capable of hybridizing, under stringent conditions and preferably under highly stringent conditions, with nucleic acid consisting of a base sequence complementary to the base sequence of SEQ ID NO: 1, and encoding protein having a $\tau m^5U$ modification activity;

(iii) nucleic acid consisting of a base sequence provided by the deletion, substitution, or addition of one or a few bases, and preferably 1, 2, 3, 4, 5, 6, 7, 8, 9, or 10 per 100 bases, in the base sequence of SEQ ID NO: 1, and encoding protein having a $\tau m^5U$ modification activity;

(iv) nucleic acid consisting of a base sequence having a sequence identity of at least 80% and preferably at least 90%, at least 91%, at least 92%, at least 93%, at least 94%, at least 95%, at least 96%, at least 97%, at least 98%, or at least 99% with SEQ ID NO: 1, and encoding protein having a $\tau m^5U$ modification activity;

(v) nucleic acid consisting of a base sequence encoding protein consisting of the amino acid sequence of SEQ ID NO: 2;

(vi) nucleic acid encoding protein having a $\tau m^5U$ modification activity and capable of hybridizing under stringent conditions, and preferably under highly stringent conditions, with nucleic acid consisting of a base sequence encoding protein consisting of the amino acid sequence of SEQ ID NO: 2;

(vii) nucleic acid consisting of a base sequence encoding protein that comprises an amino acid sequence provided by the deletion, substitution, or addition of one or a few amino acids, and preferably 1, 2, 3, 4, 5, 6, 7, 8, 9, or 10 amino acids per 100 amino acids, in the amino acid sequence of SEQ ID NO: 2, wherein the protein has a $\tau m^5U$ modification activity; or

(viii) nucleic acid consisting of a base sequence encoding protein that comprises an amino acid sequence having a sequence identity of at least 80% and preferably at least 90%, at least 91%, at least 92%, at least 93%, at least 94%, at least 95%, at least 96%, at least 97%, at least 98%, or at least 99% with SEQ ID NO: 2, wherein the protein has a $\tau m^5U$ modification activity.

The nucleic acid may be DNA or RNA, e.g., mRNA and so forth.

The vector may be a viral vector or a plasmid. The viral vector can be selected from known vectors, for example, from the group consisting of retrovirus vectors, lentivirus vectors, adenovirus vectors, and adeno-associated virus vectors. The vector may be a self-replicating vector, for example, a plasmid, an extrachromosomal element, a minichromosome, or an artificial chromosome. Viral vectors are preferred among the preceding.

The MTO1-encoding nucleic acid is operably linked to at least one control sequence for the expression of this nucleic acid. The disposition of the MTO1-encoding nucleic acid is not particularly limited as long as it is under the control of a control sequence. A promoter sequence is a control sequence. The promoter may be a constitutive promoter, a tissue-specific promoter, or an inducible promoter. The constitutive promoter includes the SV40 promoter, cytomegalovirus (CMV) promoter, and so forth.

The control sequence may additionally comprise an operator or enhancer, a mRNA ribosome binding site, and other sites that regulate the initiation of transcription or translation.

(Application of water-soluble carbodiimides)

"Water-soluble carbodiimide" means a water-soluble compound that has a carbodiimide group (-N=C=N-) in the molecule. The water-soluble carbodiimide includes N-cyclohexyl-N'-β-(4-methylmorpholinium) ethylcarbodiimide (CMC), 1-ethyl-3-(3-dimethylaminopropyl)carbodiimide (EDC), 4-(4,6-dimethoxy-1,3,5-triazin-2-yl)-4-methylmorpholinium chloride (DMT-MM), and their salts.

A compound given by the following general formula can be formed when the water-soluble carbodiimide is contacted with mitochondrial $tRNA^{Leu(UUR)}$.

[C2]

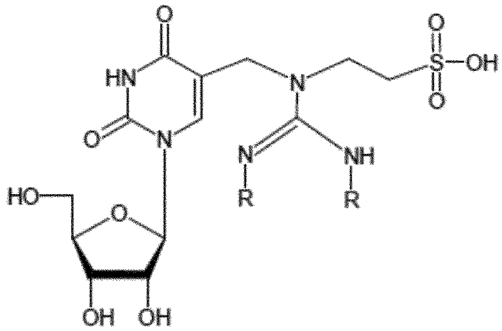

wherein R is a saturated or unsaturated hydrocarbon group, which may have a substituent, and the two R's may be the same as each other.

The "substituent" should be a functional group that can impart water solubility to the carbodiimide compound, but is not otherwise particularly limited; however, nitrogenous functional groups are preferred. Among these, nitrogenous functional groups that can form a quaternary salt are more preferred, for example, functional groups such as the N-alkylmorpholinyl group, trisubstituted amino group, and disubstituted amino group. The substituent on the amino group includes lower hydrocarbon groups, i.e., ethyl and methyl. R is preferably a substituent-bearing hydrocarbon group.

The "hydrocarbon group" in the "hydrocarbon group, which may have a substituent" includes $C_{1-19}$ hydrocarbon groups, e.g., alkyl groups, alkenyl groups, alkynyl groups, aryl groups, aralkyl groups, and so forth.

The alkyl group includes straight-chain or branched-chain $C_{1-6}$ alkyl groups, e.g., the methyl group, ethyl group, n-propyl group, isopropyl group, n-butyl group, isobutyl group, sec-butyl group, tert-butyl group, n-pentyl group, isopentyl group, sec-pentyl group, neopentyl group, n-hexyl group, and isohexyl group; and by cyclic $C_{3-14}$ alkyl groups, e.g., the cyclopentyl group and cyclohexyl group.

The alkenyl group includes straight-chain or branched-chain $C_{2-6}$ alkenyl groups, e.g., the allyl group, isopropenyl group, isobutenyl group, 2-pentenyl group, and 2-hexenyl group; and by cyclic $C_{3-14}$ alkenyl groups, e.g., the 2-cyclohexenyl group.

The alkynyl group includes straight-chain or branched-chain $C_{2-6}$ alkynyl groups, e.g., the propargyl group, 2-butynyl group, 3-butynyl group, 3-pentynyl group, and 3-hexynyl group.

The aryl group includes $C_{6-14}$ aryl groups, e.g., the phenyl group, 1-naphthyl group, 2-naphthyl group, biphenylyl group, and 2-anthryl group.

The aralkyl group includes phenyl-$C_{1-4}$ alkyl groups, e.g., the benzyl group, phenethyl group, and phenylpropyl group; and by $C_{7-19}$ aralkyl groups, e.g., the benzhydryl group and trityl group.

When the hydrocarbon group is an alkyl group, alkenyl group, or alkynyl group, the hydrocarbon group may be substituted by, for example, an alkylthio group (e.g., a $C_{1-4}$ alkylthio group, e.g., the methylthio group, ethylthio group, n-propylthio group, and isopropylthio group), a halogen atom (e.g., the fluorine atom, chlorine atom, bromine atom, and iodine atom), an alkoxy group (e.g., a $C_{1-6}$ alkoxy group, e.g., the methoxy group, ethoxy group, n-propoxy group, and isopropoxy group), an acyloxy group (e.g., a $C_{1-6}$ alkyl-carbonyloxy group, e.g., the acetyloxy group and n-propionyloxy group; a $C_{6-14}$ aryl-carbonyloxy group, e.g., the benzoyloxy group and naphthalenecarbonyloxy group), a nitro group, an alkoxycarbonyl group (e.g., a $C_{1-6}$ alkoxy-carbonyl group, e.g., the methoxycarbonyl group, ethoxycarbonyl group, n-propoxycarbonyl group, and isopropoxycarbonyl group), an alkylamino group (e.g., a mono- or di-$C_{1-6}$-alkylamino group, e.g., the methylamino group, ethylamino group, n-propylamino group, isopropylamino group, dimethylamino group, diethylamino group, methylethylamino group, and methylisobutylamino group), an alkoxyimino group (e.g., a $C_{1-6}$ alkoxyimino group, e.g., the methoxyimino group, ethoxyimino group, n-propoxyimino group, and isopropoxyimino group), or a hydroxyimino group. The number of substituents is not particularly limited, and may be, for example, 1 to 3. When

there are two or more substituents, the individual substituents may be the same or may differ.

When the hydrocarbon group is an aryl group or aralkyl group, this hydrocarbon group may be substituted by, for example, an alkyl group (e.g., a straight-chain or branched-chain $C_{1-6}$ alkyl group, e.g., the methyl group, ethyl group, n-propyl group, isopropyl group, n-butyl group, isobutyl group, sec-butyl group, tert-butyl group, n-pentyl group, and n-hexyl group; and a cyclic $C_{3-6}$ alkyl group, e.g., the cyclohexyl group), an alkenyl group (e.g., a $C_{2-6}$ alkenyl group, e.g., the allyl group, isopropenyl group, isobutenyl group, 1-methylallyl group, 2-pentenyl group, and 2-hexenyl group), an alkynyl group (e.g., a $C_{2-6}$ alkynyl group, e.g., the propargyl group, 2-butynyl group, 3-butynyl group, 3-pentynyl group, and 3-hexynyl group), an alkoxy group (e.g., a $C_{1-6}$ alkoxy group, e.g., the methoxy group, ethoxy group, n-propoxy group, and isopropoxy group), an acyl group (e.g., a $C_{1-6}$ alkyl-carbonyl group, e.g., the formyl group, acetyl group, and propionyl group; a $C_{6-14}$ aryl-carbonyl group, e.g., the benzoyl group and naphthalenecarbonyl group), a nitro group, amino group, hydroxy group, cyano group, sulfamoyl group, mercapto group, a halogen atom (e.g., the fluorine atom, chlorine atom, bromine atom, iodine atom), or an alkylthio group (e.g., a $C_{1-4}$ alkylthio group, e.g., the methylthio group, ethylthio group, n-propylthio group, and isopropylthio group). The number of substituents is not particularly limited, and may be, for example, 1 to 5. When there are two or more substituents, the individual substituents may be the same or may differ.

A compound having the following structure can be formed when mitochondrial $tRNA^{Leu(UUR)}$ is contacted with CMC as the water-soluble carbodiimide.

[C3]

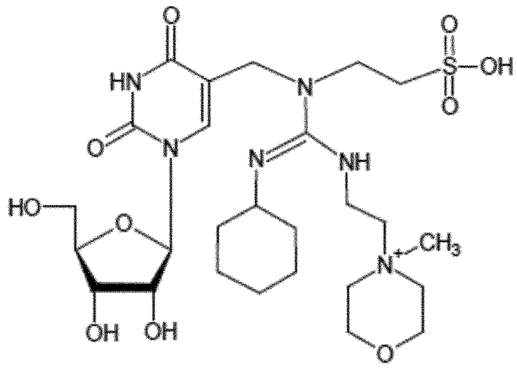

A compound having the following structure can be formed when mitochondrial $tRNA^{Leu(UUR)}$ is contacted with CMC as the water-soluble carbodiimide.

[C4]

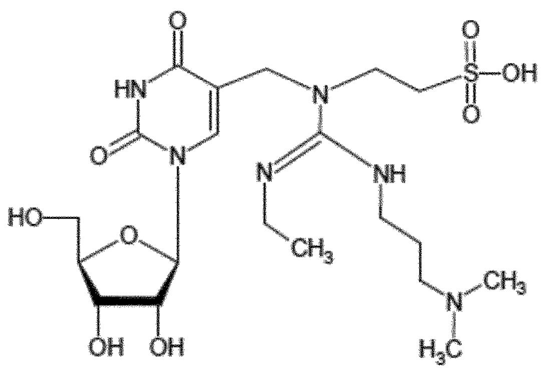

CMC, which is one of the water-soluble carbodiimides, has been used for the detection of pseudouridine (ψ) using a primer extension technique (Ofengand et al., 2001).

When a reverse transcription reaction is carried out on mitochondrial $tRNA^{Leu(UUR)}$, the reaction proceeds according to the course shown in Fig. 6

When τ$m^5$U modification is present, it is known that the reverse transcription reaction is as shown in Fig. 7,

center, and that the reverse transcription reaction stops at a position corresponding to position 33 in mitochondrial $tRNA^{Leu(UUR)}$ (Kirino et al., 2005). The present inventors discovered that when the reverse transcription reaction is carried out on a template of mitochondrial $tRNA^{Leu(UUR)}$ in the presence of a water-soluble carbodiimide, e.g., CMC, the reverse transcription reaction can be inhibited by the formation of the compound given by the general formula indicated above.

Specifically, the reverse transcription reaction is stopped at a position corresponding to position 35 in the case of $\tau m^5U$ that has been derivatized by contact with water-soluble carbodiimide (Fig. 7, right). On the other hand, when unmodified U is present, the reverse transcription reaction proceeds unimpeded and does not stop at position 33, and the extension reaction is completed by the introduction of dideoxyguanosine triphosphate (ddGTP) at the C at position 32 (Fig. 7, left). While not intending to be bound by theory, it is believed that, due to the bulkiness of the water-soluble carbodiimide-derivatized $\tau m^5U$, steric hindrance is produced to a degree that inhibits the reverse transcription reaction at the point of entry into the reverse transcriptase, and that, as a result, the reverse transcription reaction stops at a position corresponding to position 35, which is immediately before the $\tau m^5U$ modification.

The reverse transcription reaction can be carried out using a known method on a template of the $tRNA^{Leu(UUR)}$ gene and using a reverse transcriptase, a primer, and dNTPs (nucleotide mixture). As used herein, "reverse transcription reaction" means a reaction mediated by a reverse transcriptase that carries out RNA-dependent DNA synthesis. The reverse transcription reaction is divided into an annealing reaction of the primer with the template followed by an extension reaction from the primer.

Primer extension reactions, RT-PCR, and so forth are examples of reactions that use a reverse transcription reaction.

The sample used in the reverse transcription reaction is obtained by preparing an RNA-comprising sample using a conventional method. The sample may originate not only from a healthy human or a subject that is suspected of having a disease(s) caused by reduction or deficiency in $\tau m^5U$ modification, but also from, for example, non-human vertebrates (e.g., monkeys, mice, rats, hamsters, guinea pigs, rabbits, cats, dogs, pigs, bovines, horses, sheep, birds, reptiles, amphibians, fish), protochordates, mollusks, and so forth. The sample includes blood, plasma, serum, bile, saliva, urine, tears, sweat, cerebrospinal fluid, and so forth.

In some instances the mitochondrial $tRNA^{Leu(UUR)}$ derived from a subject suspected of having a disease(s) caused by reduction or deficiency in $\tau m^5U$ modification may have an A3243G mutation.

A primer that specifically hybridizes to mitochondrial $tRNA^{Leu(UUR)}$ is used in order for the mitochondrial $tRNA^{Leu(UUR)}$ present in the obtained RNA to function as the template. The primer may be an oligonucleotide complementary to the template. This oligonucleotide may be DNA, RNA, or a combinatoin of DNA and RNA. The nucleotides constituting the oligonucleotide may be natural nucleotides or artificial nucleotides.

The length of the primer is not particularly limited as long as the ability to specifically hybridize to mitochondrial $tRNA^{Leu(UUR)}$ can be retained, but is about 13 to 25 bases, for example, 13, 14, 15, 16, 17, 18, 19, 20, 21, 22, 23, 24, or 25 bases. When the primer is too short, a satisfactory hybridization efficiency is not obtained; when the primer is too long, the primer itself produces a secondary structure and/or may hybridize to another sequence, and the specificity is lost. The person skilled in the art can design the primer as appropriate.

A primer having the following sequence is an example of a DNA primer that is complementary to mitochondrial $tRNA^{Leu(UUR)}$.

ACCTCTGACTGTAAAG (SEQ ID NO: 13)

The primer may be directly or indirectly labeled with a labeling substance, and the labeling substance includes radioactive substances such as $^{32}P$, $^{35}S$, $^{14}C$, and $^{3}H$; fluorescent substances such as FITC; enzymes such as alkali phosphatase; and particles such as a metal colloid. The position of labeling is not particularly limited.

The reverse transcriptase may be freely selected; for example, various wild-type reverse transcriptases can be used, including commercially available reverse transcriptases, e.g., SuperScript (registered trademark) reverse transcriptase, M-MuLV reverse transcriptase, AMV reverse transcriptase, and MultiScribe (registered trademark) reverse transcriptase.

The dNTP may be a mixture of 4 nucleotides (for example, dATP, dGTP, dCTP, dTTP) or may be 1 to 3 nucleotides complementary to the base sequence of the template. The dNTP may optionally comprise at least one dideoxynucleotide (ddATP, ddTTP, ddCTP, ddGTP) in order to stop primer extension at a desired position. When the reverse transcription reaction is carried out in the presence of dideoxyguanosine triphosphate (ddGTP), the extension reaction can be stopped by the insertion of ddGTP at the C at position 32 of mitochondrial $tRNA^{Leu(UUR)}$.

In the absence of ddGTP, the extension reaction does not stop at position 32 and in principle can proceed to the 5'-terminal of the tRNA itself, but it is thought that in actuality extension will stop at the water-soluble carbodiimide-derivatized pseudouridine ($\psi$) at position 27 or the 1-methylguanosine ($m^1G$) at position 9.

After the completion of the reverse transcription reaction, differences in the lengths of the primer extension products due to the presence/absence of inhibition by the water-soluble carbodiimide are determined. These differences can be detected by subjecting the primer extension products to electrophoresis.

Other steps may be carried out after the completion of the reverse transcription reaction, for example, a step in which the τm⁵U modification rate is measured. The τm⁵U modification rate can be measured by a known method.

An embodiment of the present invention based on inhibition of the reverse transcription reaction by the water-soluble carbodiimide is described as an example below.

In a third embodiment, there is provided a method for detecting $\tau m^5U$ modification of mitochondrial tRNA, wherein the method comprises a step of performing, in the presence of a water-soluble carbodiimide, a reverse transcription reaction on mitochondrial $tRNA^{Leu(UUR)}$ contained in a sample derived from a subject.

In a preferred embodiment, the presence of $\tau m^5U$ modification is indicated when the reverse transcription reaction stops at a position corresponding to position 35, which is earlier than the $\tau m^5U$ at position 34 in mitochondrial $tRNA^{Leu(UUR)}$, or when the reaction stops at a position corresponding to position 33. On the other hand, the absence of $\tau m^5U$ modification is indicated when, in the presence of ddGTP, the reverse transcription reaction proceeds to the position corresponding to position 32. The absence of $\tau m^5U$ modification is indicated when, also in the absence of ddGTP, the reverse transcription reaction proceeds to or beyond position 32.

In a fourth embodiment, there is provided a method for screening for drugs that treat or prevent a disease(s) caused by reduction or deficiency in $\tau m^5U$ modification, wherein the method comprises a step of performing, in the presence of a water-soluble carbodiimide, a reverse transcription reaction on mitochondrial $tRNA^{Leu(UUR)}$ contained in a sample derived from a subject that has been treated with a candidate substance, and a step of measuring the $\tau m^5U$ modification rate.

In a preferred embodiment, when the $\tau m^5U$ modification rate is increased in comparison to that for a sample where treatment with the candidate substance was not carried out, the candidate substance is selected as a drug that treats or prevents a disease(s) caused by reduction or deficiency in $\tau m^5U$ modification.

In a fifth embodiment, there is provided a method for the in vitro detection of a disease(s) caused by reduction or deficiency in $\tau m^5U$ modification of mitochondrial tRNA, wherein the method comprises a step of performing, in the presence of a water-soluble carbodiimide, a reverse transcription reaction on mitochondrial $tRNA^{Leu(UUR)}$ contained in a sample derived from a subject.

In a preferred embodiment, it is indicated that the subject is not suffering from a disease(s) caused by reduction or deficiency in $\tau m^5U$ modification when the reverse transcription reaction stops earlier than a $\tau m^5U$ at position 34 of the mitochondrial $tRNA^{Leu(UUR)}$ or stops at a position corresponding to position 33, or
it is indicated that the subject is suffering from a disease(s) caused by reduction or deficiency in $\tau m^5U$ modification when the reverse transcription reaction progresses to or beyond the position corresponding to position 32 in the mitochondrial $tRNA^{Leu(UUR)}$.

In a sixth embodiment, there is provided a method for assessing whether a subject that has, or is suspected of having, a disease(s) caused by reduction or deficiency in $\tau m^5U$ modification is a target for treatment of the disease using, e.g., taurine and so forth, wherein this method comprises a step of performing, in the presence of a water-soluble carbodiimide, a reverse transcription reaction on mitochondrial $tRNA^{Leu(UUR)}$ contained in a sample derived from a subject.

In a preferred embodiment, it is indicated that the subject can be a target for a treatment of the disease using, e.g., taurine and so forth, when the reverse transcription reaction stops earlier than a $\tau m^5U$ at position 34 of the mitochondrial $tRNA^{Leu(UUR)}$ or stops at a position corresponding to position 33, or
it is indicated that the subject can be a target for a treatment of the disease using, e.g., taurine and so forth, when the reverse transcription reaction progresses to or beyond the position corresponding to position 32 in the mitochondrial $tRNA^{Leu(UUR)}$.

(Nucleic acid bonded to water-soluble carbodiimide)

In a seventh embodiment, there is provided a compound having the following structure or a salt thereof:

[C5]

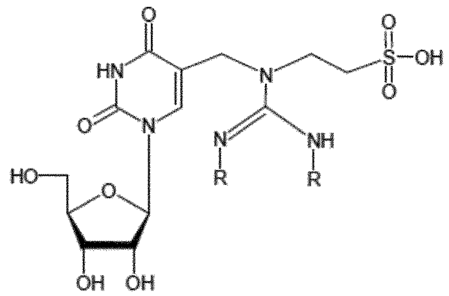

wherein R is a saturated or unsaturated hydrocarbon group, which may have a substituent, and the two R's may be the same as each other.

The "substituent" should be a functional group that can impart water solubility to the carbodiimide compound, but is not otherwise particularly limited; however, nitrogenous functional groups are preferred. Among these, nitrogenous functional groups that can form a quaternary salt are more preferred, for example, functional groups such as the N-alkylmorpholinyl group, trisubstituted amino group, and disubstituted amino group. The substituent on the amino group includes lower hydrocarbon groups, i.e., ethyl and methyl. R is preferably a substituent-bearing hydrocarbon group. R is defined as above.

The compound has the following structure when the water-soluble carbodiimide is CMC.

[C6]

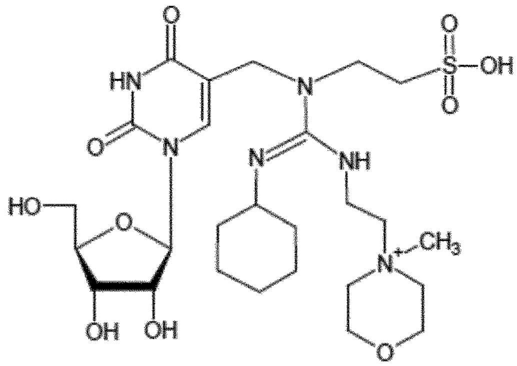

The compound has the following structure when the water-soluble carbodiimide is EDC.

[C7]

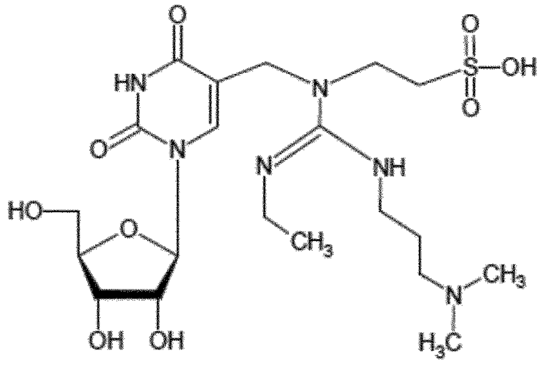

In an eighth embodiment, there is provided an inhibitor of a reverse transcription reaction on $\tau m^5U$ modification-comprising nucleic acid, wherein the inhibitor comprises a water-soluble carbodiimide as an active ingredient.

Besides $\tau m^5U$ modification-comprising nucleic acid, it is thought that the reverse transcription reaction inhibitor can also be used for other uridine derivatives that have a skeleton similar or analogous to $\tau m^5U$ modification-comprising nucleic acid.

The definition of this water-soluble carbodiimide is as above.

In a ninth embodiment, there is provided a kit comprising a reverse transcriptase and an inhibitor of a reverse transcription reaction on $\tau m^5U$ modification-comprising nucleic acid, wherein the inhibitor comprises a water-soluble carbodiimide as an active ingredient.

A freely selected reverse transcriptase can be used for the reverse transcriptase; for example, various wild-type reverse transcriptases can be used, including commercially available reverse transcriptases, e.g., SuperScript (registered trademark) reverse transcriptase, M-MuLV reverse transcriptase, AMV reverse transcriptase, and MultiScribe (registered trademark) reverse transcriptase, with SuperScript (registered trademark) III reverse transcriptase being preferred.

The present invention is more specifically described in the following examples, but the present invention is not limited to or by these.

## Examples

1. Construction of MTO1 expression vector

Homo sapiens-originating mitochondrial tRNA translation optimization 1 (MTO1) isoform a (NP_036255.2, NM_012123.4, MTO1 in the following) having the FLAG tag fused to the C-terminal (MTO1_FLAG in the following) was introduced into a lentivirus vector (Fig. 1A).

Using as template the vector pDEST_MTO1 (Asano et al., 2018) in which MTO1 cDNA was cloned, the MTO1 cDNA moiety was amplified by PCR (PCR reaction condition 1) using Fw primer 1 (SEQ ID NO: 3: acctccatagaagacaccgactctagaggatccaccggtcgccaccATGttctacttccgaggctg) and Rv primer 1 (SEQ ID NO: 4: cggccgctttaTTTATCATCATCATCTTTATAATCTCCTCCtaactctctctcttgaagtctgtctg).

PCR reaction condition 1

1X PCR buffer for KOD-Plus-Neo

0.2 mM dNTP

1.5 mM $MgSO_4$

0. 25 pmol/μL Fw primer 1

0. 25 pmol/μL Rv primer 1

8.4 ng/μL pDEST_MTO1

0.02 U/μL KOD-Plus-Neo

[Table 1]

| | |
|---|---|
| 94 °C 2 min | |
| 98 °C 10 sec | ×24 cycle |
| 58 °C 30 sec | ×24 cycle |
| 68 °C 1.5 min | ×24 cycle |

(continued)

4 °C

In addition, using pLenti CMV GFP Puro as template, the vector sequence required for cloning was amplified by PCR (PCR reaction condition 2) using Fw primer 2 (SEQ ID NO: 5: agtcagtccccgggggaggaGATTATAAAGATGATGATG) and Rv primer 2 (SEQ ID NO: 6: agtcagtcGATATCgaccggtggatcctctag).

PCR reaction condition 2

1X PrimeSTAR buffer

0.2 mM dNTP

0. 32 pmol/μL Fw primer 2

0. 32 pmol/μL Rv primer 2

5 ng/μL pLenti CMV GFP Puro

0.05 U/μL PrimeSTAR DNA polymerase

[Table 2]

| | |
|---|---|
| 94 °C 2min | |
| 98 °C 10sec | ×17 cycle |
| 55 °C 5 sec | |
| 72 °C 10min | |
| 4 °C | |

After the PCR reaction, the template DNA was removed by adding DpnI (TAKARA) to each PCR product and incubating overnight at 37°C. This was followed by purification of the PCR product using a FastGene column (Nippon Genetics Co., Ltd.) and reaction (SLiCE reaction conditions) using the SLiCE method (Okegawa et al., 2015).

SLiCE reaction conditions (10 μL reaction system)

50 mM Tris-HCl (pH 7.5)

10 mM $MgCl_2$

1 mM ATP

1 mM DTT

1 μL SLiCE extract (S100 fraction of JM109)

10 ng linear vector

50 ng insert DNA

DH5a cells were then transformed with the reaction product and cultured overnight at 37°C on LB agar culture medium comprising ampicillin. The grown colonies were inoculated onto liquid LB culture medium comprising ampicillin and the culture was shaken overnight at 37°C. Plasmid was extracted from the cultured cells, and a pLenti vector into which the cDNA of the target MTO1_FLAG (below, pLenti_MT01_FLAG vector) had been inserted was confirmed by DNA sequence analysis. The MTO1_FLAG gene encoded in pLenti_MT01_FLAG is transcribed under control by the CMV promoter (Fig. 1A).

2. Preparation of a stable MTO1-overexpressing cell line

Using a lentivirus system, the MTO1_FLAG gene was inserted into the genome of myoblasts derived from a MELAS patient (Sasarman et al., 2008) to construct a stable MTO1-FLAG-expressing cell line. To this end, a lentivirus expressing the MTO1_FLAG gene was first constructed using the following procedure. 2 μg of pLenti MTO1_FLAG, 7.5 μg of pMDLg/pRRE (Addgene #12251), 7.5 μg of pRSV/REV (Addgene #12253), and 5 μg of pMD2.G (Addgene #12259) were mixed with 176 μL of polyethyleneimine (PEI) and 2 mL of Opti-MEM (registered trademark) I Reduced Serum Medium (Gibco) and left for 20 minutes at room temperature. Transfection was carried out by adding the mixture to HEK293T cells ($4 \times 10^6$ cells) that had been seeded to a culture Petri dish (15 cm) on the previous day.

The HEK293T cells were cultured for 8 hours at 37°C under 5% $CO_2$ in DMEM culture medium in the presence of 5% FBS, followed by exchange for fresh culture medium and collection of the culture medium after culture for 3 days. Fresh culture medium was added, culture was continued, and the culture medium was collected on the following day. The target packaged lentivirus is released into the culture supernatant. The recovered culture supernatant was filtered on a filter (0.22 μm) and the fraction passing through was subjected to ultracentrifugal separation for 1 hour at 35,000 × g to recover the target lentivirus by sedimentation.

Myoblasts derived from a MELAS patient were transduced with the obtained lentivirus. A culture medium, which is provided by the addition of 1X penicillin-streptomycin solution (Wako), 10 mM taurine, and 50 μg/mL uridine to SkGM (registered trademark)-2 BulletKit (registered trademark) (Lonza), was used as the culture medium for culturing the myoblasts. After several days, the cells were subcultured in the medium comprising 2 ug/mL of puromycin for the selection of puromycin-resistant cells, and the grown cell colonies were isolated to obtain a stable expressing cell line. MTO1_FLAG expression within the cells and mitochondrial localization were confirmed by Western blotting and immunostaining for the FLAG tag (Fig. 1B). In addition, a stable GTPBP3-expressing cell line was also constructed for comparison. A stable green fluorescent protein (eGFP)-expressing cell line was constructed as a control cell line.

2.1 Confirmation of expression by RT-qPCR

The total RNA was extracted from each isolated cell line and the amount of mRNA was measured by RT-qPCR using the following protocol.

The admixed DNA was degraded by holding the reaction solution (10 μL), which contained 1 μg of total RNA, 1 μL of 10X Reaction Buffer [400 mM Tris-HCl (pH 8.0), 100 mM $MgSO_4$, 10 mM $CaCl_2$] (Promega), and 1 μL of 1 U/mL RQ1 RNase-Free DNase (Promega), for 30 minutes at 37°C. After this, the reaction was stopped by adding 1 μL of Stop Buffer [20 mM EGTA (pH 8.0)] (Promega) and holding for 10 minutes at 65°C. cDNA was synthesized using a First Strand cDNA Synthesis Kit for RT-PCR (Roche), and 80 μL of ultrapure water was added. To 2.5 μL of the cDNA solution was added 7.5 μL of Premix [1 μL each of Fw primer and Rv primer, 48 μL of ultrapure water, and 100 μL of 2X KAPA SYBR FAST qPCR Master Mix (Roche)], and measurement was carried out under the following reaction conditions using a thermal cycler (Lightcycler 96, Roche).

[Table 3]

| | Reaction Conditions | |
|---|---|---|
| 95°C | 3 min | |
| 95°C | 10s | |
| 57°C | 20s | ×40 (57°C 20s through 72°C 1s) |
| 72°C | 1s | |
| 95°C | 5s | |
| 65°C | 60s | |
| 97°C | 1s | |

Primer sequences

| | | |
|---|---|---|
| MTO1 | Fw: | AAACAAGCATATACCGGACAATC (SEQ ID NO: 7) |
| | Rv: | TTGATTCAATGGAGGGACAGTAT (SEQ ID NO: 8) |
| GTPBP3 | Fw: | GTTTCACCGGTGAGGACTG (SEQ ID NO: 9) |
| | Rv: | TGTTTCCGCGTGGATAAGGT (SEQ ID NO: 10) |
| GAPDH | Fw: | GAGCCAAAAGGGTCATCATC (SEQ ID NO: 11) |
| | Rv: | CCATCACGCCACAGTTTCC (SEQ ID NO: 12) |

Based on the RT-qPCR results for the MTO1- and GTPBP3-overexpressing cell lines, relative values were calculated versus the MTO1 and GTPBP3 mRNA in the MELAS_GFP cell line that is the control cell line. In addition, normalization with GAPDH mRNA as internal standard was also performed (Fig. 2). With regard to the MTO1 cell line, the steady-state level of MTO1 mRNA was increased 18.5- to 50.0-fold in comparison to the MELAS_GFP cell line. With regard to the GTPBP3 cell line, the mRNA was increased 31.7-fold in comparison to the MELAS_GFP cell line.

3. The CMC-PE technique

CMC (N-cyclohexyl-N'-β-(4-methylmorpholinium) ethylcarbodiimide) has been used for the detection of pseudouridine (ψ) using a PE technique (Ofengand et al., 2001). The present inventors discovered that, when $\tau m^5U$ has been derivatized with CMC (Fig. 3A), this stops the reverse transcription reaction at position 35 and position 33, and that with unmodified U the reverse transcription reaction proceeds and the extension reaction is completed by the introduction of dideoxyguanosine triphosphate (ddGTP) at the C of position 32 (Fig. 3B). In addition, quantitative measurement of a $\tau m^5U$ modification rate was made possible by quantitating the values of these three bands and using the values for the bands at position 33 and position 35 that participate in the total value of these bands. The band intensity ratio was calculated using the following formula.

band intensity ratio = (band intensity for position 33 + band intensity for position 35)/(total value of band intensities for position 32, position 33, and position 35)

In the following, the PE technique using CMC is also referred to as the CMC-PE technique, but the same results as for CMC are also obtained when water-soluble carbodiimides other than CMC are used.

· CMC derivatization of RNA

Using the acid guanidinium thiocyanate phenol chloroform extraction (AGPC) method (Chomczynski and Sacchi, 1987), the total RNA was extracted from the cells obtained as described above. A 15 μg fraction of the total RNA was dried up and dissolved in 15 μL of BEU buffer [50 mM bicine (pH 8.3), 4 mM EDTA (pH 8.0), 7 M urea]. 15 μL of BEU buffer in 0.34 M CMC-p-toluenesulfonate was added and mixed thoroughly, and a reaction was carried out for 20 minutes at 37°C. This was followed by the addition of 100 μL of stop solution [0.3 M NaOAc (pH 5.2), 0.1 mM EDTA (pH 8.0)] and 700 μL of ethanol and mixing, followed by recovery of the CMC-derivatized RNA by ethanol precipitation. Then, after rinsing with 70% ethanol, purification was carried out by dissolving the pellet in 100 μL of stop solution and reprecipitating with ethanol by adding 300 μL of ethanol. The purified RNA was vacuum dried and dissolved in 40 μL of 50 mM $Na_2CO_3$ (pH 10.4) and a reaction was carried out for 4 hours at 37°C to remove the CMC bonded to the uracil ring or guanine ring. To reaction solution was added 100 μL of stop solution for neutralization and the RNA was recovered by ethanol precipitation by adding 700 μL of ethanol and was dissolved in 10 μL of ultrapure water.

· Labeling of the 5'-terminal of the primer

$^{32}P$-labeling of the 5'-terminal of the primer was performed by carrying out a reaction for 1 hour at 37°C in a reaction solution (10 μL) that contained 4 pmol (2 μL) of a DNA primer (SEQ ID NO: 13: ACCTCTGACTGTAAAG) complementary to mitochondrial $tRNA^{Leu(UUR)}$, 1 μL of [$\gamma^{32}P$]-ATP (PerkinElmer Inc.), 1 μL of 10X Protruding End Kinase Buffer (Toyobo), 0.5 μL of T4 Polynucleotide Kinase (Toyobo), and 5.5 μL of ultrapure water. This was followed by deactivation of the enzyme by heating for 15 minutes at 65°C, after which the $^{32}P$-labeled DNA primer was submitted to gel filtration purification using a CENTRI-SEP spin column (Princeton Separations). The radioactivity was measured

using Cerenkov light.

· Reverse transcription reaction

A solution (5 μL) comprising 0.75 μg of the CMC-derivatized total RNA, 0.05 pmol (approximately 50,000 cpm) of the $^{32}P$-labeled DNA primer, 1 mM EDTA-NaOH (pH 8.0), and 10 mM Tris-HCl (pH 7.7) was heated for 2 minutes at 80°C, followed by gradual cooling to room temperature to hybridize the primer to the mitochondrial $tRNA^{Leu(UUR)}$ in the total RNA. To this reaction solution were added 1.5 μL of 25 mM $MgCl_2$, 0.25 μL of d/dd NTP mix (1.5 mM dATP, 1.5 mM dTTP, 3.0 mM ddGTP), 2 μL of 5X FS buffer, 0.5 μL of SuperScript III reverse transcriptase (Invitrogen), and 0.75 μL of ultrapure water, an extension reaction was carried out for 1 hour at 55°C; and incubated for 15 minutes at 70°C to deactivate the enzyme. After the reaction, 0.5 μL of 4M NaOH was added and the RNA was degraded by heating for 5 minutes at 95°C. The reaction was neutralized by addition of 14.5 μ L of Loading Solution (12.5 μ L of 2X Loading Solution for Urea PAGE, 1 μ L of 1 M Tris, and 1 μ L of 1 M HCl) and incubation for 5 minutes at 65°C.

· Quantitative determination of the amount of cDNA

The reaction product was separated using denaturing polyacrylamide gel electrophoresis (20% PAGE with 7 M urea, 20 × 20 $cm^2$, 0.35 mm thickness), the gel was exposed to an imaging plate (BAS-MS2040, Fujifilm) overnight (-30°C), and the radiolabeled bands were detected using an image analyzer (FLA-7000, Fujifilm). The detected bands were quantitated using a MultiGauge.

4. Detection of $\tau m^5U$ modification using the CMC-PE technique

In order to examine the quantitative character of the CMC-PE technique, CMC-PE was carried out using RNA extracted from HeLa cells and from GTPBP3 knockout cells (G3KO) (Fig. 3B). It has been demonstrated by LC/MS analysis of mitochondrial $tRNA^{Leu(UUR)}$ isolated and purified from each of these cells that the $\tau m^5U$ modification rates in G3KO cells and in HeLa cells are 0% and 96.3%, respectively. Based on this, and using samples provided by mixing these two types of total RNAs at ratios of 0:1, 1:3, 2:2, 3:1, and 1:0, the band intensity ratio and the $\tau m^5U$ modification rate were associated by constructing a calibration curve between the band intensity ratio calculated by carrying out CMC-PE according to the method described above and the $\tau m^5U$ modification rate calculated from the mixing ratio of the two samples (Fig. 3C). Linearity in modification rate measurement using the PE technique was confirmed from the calibration curve.

The $\tau m^5U$ modification rate was quantitatively determined based on this calibration curve for the stable MTO1-expressing cell line, stable GTPBP3-expressing cell line, and stable eGFP-expressing cell line. It was found that, versus a $\tau m^5U$ modification rate of 15.5% in the stable eGFP-expressing cell line, the $\tau m^5U$ modification rate was increased to 26.7% in the stable GTPBP3-expressing cell line and to 95.3% in the stable MTO1-expressing cell line (Figs. 3D, 3E).

5. Analysis of steady-state level of MELAS mutant mitochondrial tRNA using Northern blotting

Then, Northern blotting analysis was performed to investigate whether the steady-state level of MELAS mutant mitochondrial tRNA is changed by overexpression of MTO1 in MELAS cells. 5S rRNA was used for the control.

5 μg of total RNA was separated by denaturing polyacrylamide gel electrophoresis (10% PAGE with 7 M urea). A nylon membrane (Hybond-N+, GE Healthcare) was immersed in TBE buffer (44.5 mM Tris, 44.5 mM borate, 2 mM EDTA), the gel was stacked on this, filter paper immersed in TBE was stacked four above and four below this, a voltage of 1.5 $mA/cm^2$ was applied, and electroblotting was performed for 35 minutes. The membrane was then dried and was irradiated twice with UV at 120 $mJ/cm^2$ to crosslink the RNA to the membrane. The membrane was immersed in a hybridization buffer (PerfectHyb (registered trademark) Plus, Sigma) and was held for at least 30 minutes at 50°C. DNA probes complementary to, respectively, mitochondrial $tRNA^{Leu(UUR)}$ and 5S rRNA (mitochondrial $tRNA^{Leu(UUR)}$: TGTTAAGAAGAGGAATTGAACCTCTGACTG (SEQ ID NO: 14), 5S rRNA: GGGTGGTATGGCCGTAGAC (SEQ ID NO: 15)) were labeled with $^{32}P$ using the same procedure as for the primer in the CMC-PE method and were added to the hybridization buffer and incubated overnight at 50 °C.

The membrane was lightly washed with 1X SSC [150 mM NaCl, 15 mM sodium citrate (pH 7.0)] and then shaken for 10 minutes with 1X SSC 3 times. Printing to an imaging plate was carried out for about 1 hour and the radiolabeled bands were detected using an image analyzer.

As previously reported (Yasukawa et al., 2000), the steady-state level of mitochondrial $tRNA^{Leu(UUR)}$ was significantly reduced in a stable eGFP-expressing cell line (MELAS myoblasts) in comparison to a wild-type cell line (Figs. 4A and 4B). In comparison to the stable eGFP-expressing cell line, it was found that the steady-state level of mitochondrial $tRNA^{Leu(UUR)}$ was not changed in the stable GTPBP3-expressing cell line, but was significantly increased

in the stable MTO1-expressing cell line (Figs. 4A and 4B). Based on these results, it was found that not only does the overexpression of MTO1 have the effect of increasing the τm5U modification rate of MELAS mutant mitochondrial $tRNA^{Leu(UUR)}$, it also has the effect of increasing the steady-state level.

6. Evaluation of the protein synthesis capability by a pulse-labeling technique

The mitochondrial protein synthesis was evaluated by stopping cytoplasmic translation reactions using the antibiotic emetine and carrying out pulse-labeling with [$^{35}$S]-methionine and -cysteine to specifically label mitochondrial translation products.

Myoblasts from healthy individuals, the stable eGFP-expressing cell line, and the stable MTO1-overexpressing cell line were seeded at $4.0 \times 10^5$ cells to collagen-coated 6-well plates and were cultured overnight at 37°C. The culture medium was removed; washed with PBS twice; and replaced with DMEM-high glucose, no glutamine, no methionine, no cystine (#210313024, Gibco) comprising 10% dialyzed FBS, 10 mM taurine, and 2 mM L-glutamine; and cultured for 10 minutes at 37°C. Emetine (final concentration = 50 μg /mL) was added to this; cultured at 37°C for 10 minutes; this was followed by the addition of 0.2 mCi [$^{35}$S] methionine and cysteine (EXPRE$^{35}$S$^{35}$S Protein Labeling Mix, [$^{35}$S] methionine/cysteine, PerkinElmer, Inc.); and cultured for an additional 1 hour. The medium was changed to DMEM-high glucose (D5796, Sigma-Aldrich) comprising 10% dialyzed FBS and 10 mM taurine and cultured at 37°C for 10 minutes. The culture medium was removed, washing with PBS was done twice, and the cells were collected with 50 μL of *Lysis buffer. The lysates were shaken for 30 minutes at 4°C and 13,000 rpm, centrifuged for 20 minutes at 4°C and 15,000 rpm, and the supernatant was collected.

*Lysis buffer: RIPA + EDTA buffer [50 mM Tris-HCl (pH 7.5), 150 mM NaCl, 1% NP-40, 0.5% sodium deoxycholate, 0.1% SDS, 1 mM EDTA-NaOH (pH 8.0)], 1 mM DTT, and 1X protease inhibitor (complete Protease Inhibitor Cocktail (#04693116001, Roche) dissolved in 2 mL of ultrapure water to prepare 25-fold concentrated stock solution)]

SDS loading solution was added to 30 μg of total protein, followed by denaturation with SDS by incubating for 30 minutes at 40°C, and separation was carried out with Tricine PAGE. The gel was washed three times with ultrapure water and stained with CBB stain one ((#04543-51, Nacalai Tesque, Inc.) for 1 hour. After destaining by washing with ultrapure water several times, the gel was shaken for 10 minutes in an aqueous solution comprising 20% methanol and 2% glycerol. The gel was thoroughly dried for 2 hours at 60°C using a gel dryer (AE-3750 RapiDry, ATTO) set to a temperature gradient of 1°C/min; this was exposed to an imaging plate for about 3 days and detection of the radiolabeled bands using an image analyzer.

With the stable eGFP-expressing cell line, the mitochondrial protein synthesis capability was globally reduced in comparison to the wild type. It was shown that, for the stable MTO1-expressing cell line, the mitochondrial protein synthesis capability was increased relative to the stable eGFP-expressing cell line (Fig. 5).

The preceding results demonstrate that, by overexpression of MTO1 in MELAS patient cells, the τm5U modification rate of MELAS mutant mitochondrial $tRNA^{Leu(UUR)}$ can be increased and mitochondrial function can be stimulated.

Literature

Suzuki, T., The expanding world of tRNA modifications and their disease relevance, Nature Reviews Molecular Cell Biology 22 (6), 375-392 (2021).

Suzuki, T., RNA modification and biological phenomenon, Journal of Japanese Biochemical Society 92(3): 431-446 (2020).

Asano, K., Suzuki, T., Saito, A., Wei, F., Ikeuchi, Y., Numata, T., Tanaka, R.,

Yamane, Y., Yamamoto, T., Goto, T., Kishita, Y., Murayama, K., Ohtake, A.,

Okazaki, Y., Tomizawa, K., Sakaguchi, Y. and Suzuki, T. Metabolic and chemical regulation of tRNA modification associated with taurine deficiency and human disease. Nucleic Acids Res. 46, 1565-1583 (2018).

Okegawa, Y. and Motohashi, K. A simple and ultra-low cost homemade seamless ligation cloning extract (SLiCE) as an alternative to a commercially available seamless DNA cloning kit. Biochem. Biophys. Rep. 4, 148-151 (2015).

Sasarman, F., Antonika, H., and Shoubridge, A., E. The A3243G tRNALeu(UUR) MELAS mutation causes amino acid misincorporation and a combined respiratory chain assembly defect partially suppressed by overexpression of EFTu and EFG2. Hum. Mol. Genet. 17, 3697-3707 (2008).

Kirino, Y., Goto, Y., Campos, Y., Arenas, J., & Suzuki, T. "Specific correlation between the wobble modification deficiency in mutant tRNAs and the clinical features of a human mitochondrial disease." PNAS. 102, 7127-7132 (2005).

Ofengand, J., Del Campo, M. & Kaya, Y. Mapping pseudouridines in RNA molecules. Methods 25, 365-373 (2001).

Chomczynski, P. and Sacchi, N. Single-step method of RNA isolation by acid guanidinium thiocyanate-phenol-chloroform extraction. Anal Biochem 162, 156-159 (1987).

Yasukawa, T., Suzuki, T., Suzuki, T., Ueda, T., Ohta, S. and Watanabe, K. Modification Defect at Anticodon Wobble Nucleotide of Mitochondrial tRNAsLeu(UUR) with Pathogenic Mutations of Mitochondrial Myopathy, Encephalopathy, Lactic Acidosis, and Stroke-like Episodes. Journal of Biological Chemistry 275, 4251-4257 (2000).

## Claims

1. A pharmaceutical composition for the treatment or prevention of a disease(s) caused by reduction or deficiency in 5-taurinomethyluridine ($\tau m^5U$) modification of mitochondrial tRNA, the composition comprising mitochondrial tRNA translation optimization 1 (MTO1) or nucleic acid encoding MTO1, wherein MTO1 is administered to a patient in an excess amount, or MTO1 is overexpressed in a patient to whom the nucleic acid has been administered.

2. The pharmaceutical composition according to claim 1, wherein a $\tau m^5U$ modification rate is increased by the administration of MTO1 or the nucleic acid, in comparison to that prior to the administration.

3. The pharmaceutical composition according to claim 2, wherein a $\tau m^5U$ modification rate is increased in comparison to the administration of excess GTP-binding protein 3 (GTPBP3) or in comparison to the overexpression of nucleic acid encoding GTPBP3.

4. The pharmaceutical composition according to any one of claims 1 to 3, wherein the nucleic acid encoding MTO1 has:

   (i) nucleic acid consisting of a base sequence of SEQ ID NO: 1;
   (ii) nucleic acid capable of hybridizing under stringent conditions with nucleic acid consisting of a base sequence complementary to the base sequence of SEQ ID NO: 1, and encoding protein having a $\tau m^5U$ modification activity;
   (iii) nucleic acid consisting of a base sequence provided by deletion, substitution, or addition of one or a few bases in the base sequence of SEQ ID NO: 1, and encoding protein having a $\tau m^5U$ modification activity;
   (iv) nucleic acid consisting of a base sequence having an at least 80% sequence identity with SEQ ID NO: 1, and encoding protein having a $\tau m^5U$ modification activity;
   (v) nucleic acid consisting of a base sequence encoding protein consisting of the amino acid sequence of SEQ ID NO: 2;
   (vi) nucleic acid capable of hybridizing under stringent conditions with nucleic acid consisting of a base sequence encoding protein consisting of the amino acid sequence of SEQ ID NO: 2, and encoding protein having a $\tau m^5U$ modification activity;
   (vii) nucleic acid consisting of a base sequence encoding protein consisting of an amino acid sequence provided by deletion, substitution, or addition of one or a few amino acids in the amino acid sequence of SEQ ID NO: 2, with the protein having a $\tau m^5U$ modification activity; or
   (viii) nucleic acid consisting of a base sequence encoding protein consisting of an amino acid sequence having a sequence identity of at least 80% with SEQ ID NO: 2, with the protein having a $\tau$m5U modification activity.

5. The pharmaceutical composition according to any one of claims 1 to 4, wherein the nucleic acid encoding MTO1 is contained in a vector.

6. The pharmaceutical composition according to claim 5, wherein the vector is a viral vector or a plasmid.

7. The pharmaceutical composition according to claim 6, wherein the viral vector is selected from the group consisting

of retrovirus vectors, lentivirus vectors, adenovirus vectors, and adeno-associated virus vectors.

**8.** The pharmaceutical composition according to any one of claims 1 to 7, wherein the MTO1 or nucleic acid encoding MTO1 is administered systemically.

**9.** The pharmaceutical composition according to any one of claims 1 to 8, wherein the disease(s) caused by reduction or deficiency in $\tau m^5U$ modification is mitochondrial encephalomyopathy.

**10.** A vector comprising nucleic acid encoding MTO1.

**11.** A method of detecting $\tau m^5U$ modification of mitochondrial tRNA, the method comprising:

a step of performing, in the presence of a water-soluble carbodiimide, a reverse transcription reaction on mitochondrial $tRNA^{Leu(UUR)}$ contained in a sample derived from a subject, wherein
the presence of $\tau m^5U$ modification is indicated when the reverse transcription reaction stops earlier than a $\tau m^5U$ at position 34 of the mitochondrial $tRNA^{Leu(UUR)}$ or stops at a position corresponding to position 33, or
the absence of $\tau m^5U$ modification is indicated when the reverse transcription reaction progresses to or beyond a position corresponding to position 32.

**12.** The method according to claim 11, wherein, when $\tau m^5U$ modification is present, the reverse transcription reaction stops at a position of adenosine at position 35 or a position of uridine at position 33.

**13.** The method according to claim 11 or 12, wherein the reverse transcription reaction is a primer extension reaction using a reverse transcriptase.

**14.** The method according to any one of claims 11 to 13, wherein the method further comprises a step of measuring a $\tau m^5U$ modification rate.

**15.** The method according to any one of claims 11 to 14, wherein the mitochondrial $tRNA^{Leu(UUR)}$ has an A3243G mutation.

**16.** The method according to any one of claims 11 to 15, wherein the water-soluble carbodiimide is selected from the group consisting of N-cyclohexyl-N'-β-(4-methylmorpholinium) ethylcarbodiimide (CMC), 1-ethyl-3-(3-dimethylaminopropyl) carbodiimide (EDC), 4-(4,6-dimethoxy-1,3,5-triazin-2-yl)-4-methylmorpholinium chloride (DMT-MM), and salts thereof.

**17.** A method of screening for drugs that treat or prevent a disease(s) caused by reduction or deficiency in $\tau m^5U$ modification, the method comprising:

a step of performing, in the presence of a water-soluble carbodiimide, a reverse transcription reaction on mitochondrial $tRNA^{Leu(UUR)}$ contained in a sample derived from a subject that has been treated with a candidate substance; and
a step of measuring a $\tau m^5U$ modification rate,
wherein, when the $\tau m^5U$ modification rate is increased in comparison to that for a sample where treatment with the candidate substance has not been carried out, the candidate substance is selected as a drug that treats or prevents a disease(s) caused by reduction or deficiency in $\tau m^5U$ modification.

**18.** A method for in vitro detection of a disease(s) caused by reduction or deficiency in $\tau m^5U$ modification of mitochondrial tRNA, the method comprising:

a step of performing, in the presence of a water-soluble carbodiimide, a reverse transcription reaction on mitochondrial $tRNA^{Leu(UUR)}$ contained in a sample from a subject, wherein
it is indicated that the subject is not suffering from a disease(s) caused by reduction or deficiency in $\tau m^5U$ modification when the reverse transcription reaction stops earlier than a $\tau m^5U$ at position 34 of the mitochondrial $tRNA^{Leu(UUR)}$ or stops at a position corresponding to position 33, or
it is indicated that the subject is suffering from a disease(s) caused by reduction or deficiency in $\tau m^5U$ modification when the reverse transcription reaction progresses to or beyond a position corresponding to position 32 in the mitochondrial $tRNA^{Leu(UUR)}$ .

**19.** A method for determining whether or not a subject that has or is suspected of having a disease(s) caused by reduction or deficiency in τm5U modification is to be a target for treatment with taurine, the method comprising:

a step of performing, in the presence of a water-soluble carbodiimide, a reverse transcription reaction on mitochondrial $tRNA^{Leu(UUR)}$ contained in a sample derived from a subject, wherein
it is indicated that the subject can be a target for a treatment using taurine, when the reverse transcription reaction stops earlier than a $\tau m^5U$ at position 34 of the mitochondrial $tRNA^{Leu(UUR)}$ or stops at the position corresponding to position 33, or
it is indicated that the subject can be a target for a treatment using taurine when the reverse transcription reaction progresses to or beyond the position corresponding to position 32 of the mitochondrial $tRNA^{Leu(UUR)}$.

**20.** A compound having a structure below or a salt thereof:

[C1]

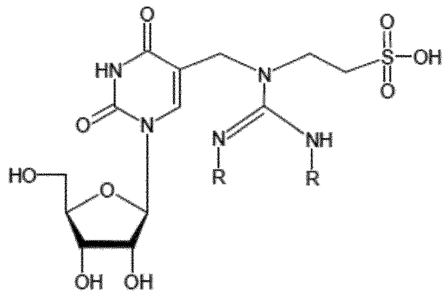

wherein R is a saturated or unsaturated hydrocarbon group, which may have a substituent, and two R's may be the same as each other.

**21.** An inhibitor of a reverse transcription reaction on $\tau m^5U$ modification-comprising nucleic acid, the inhibitor comprising a water-soluble carbodiimide as an active ingredient.

**22.** A kit comprising a reverse transcriptase and the reverse transcription reaction inhibitor according to claim 21.

Fig. 1

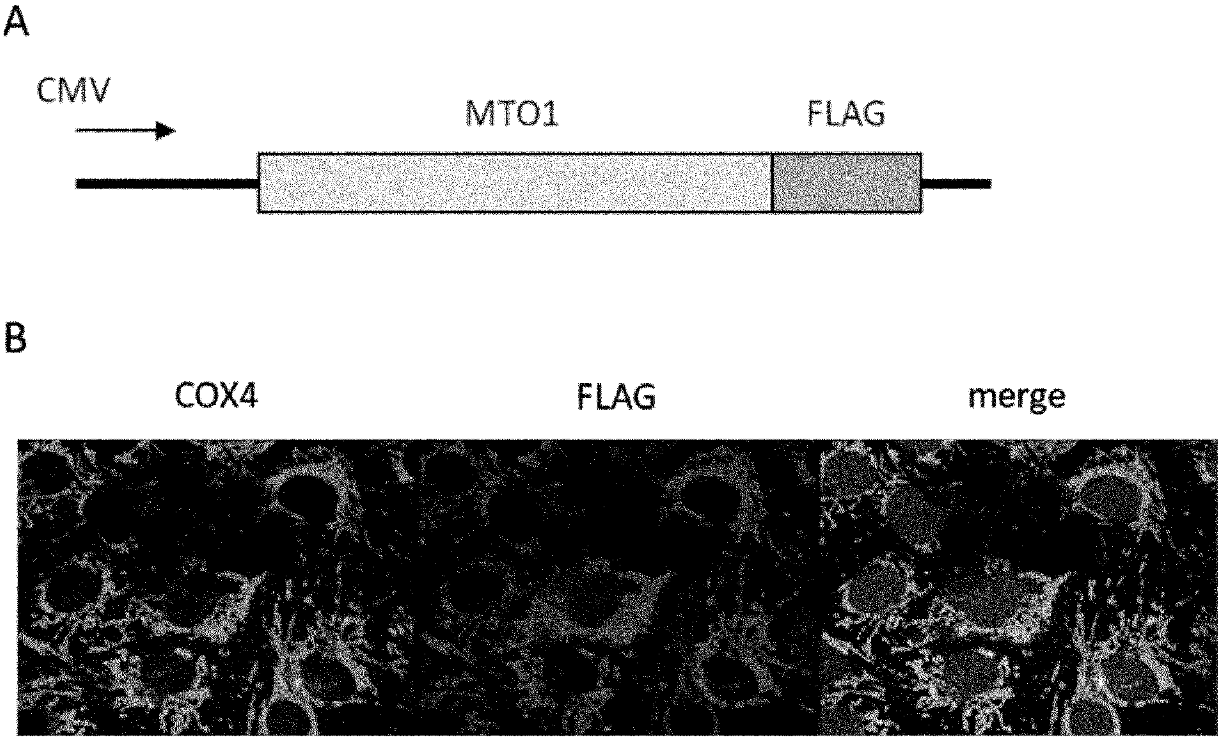
A
CMV
MTO1
FLAG
B
COX4
FLAG
merge

Fig. 2

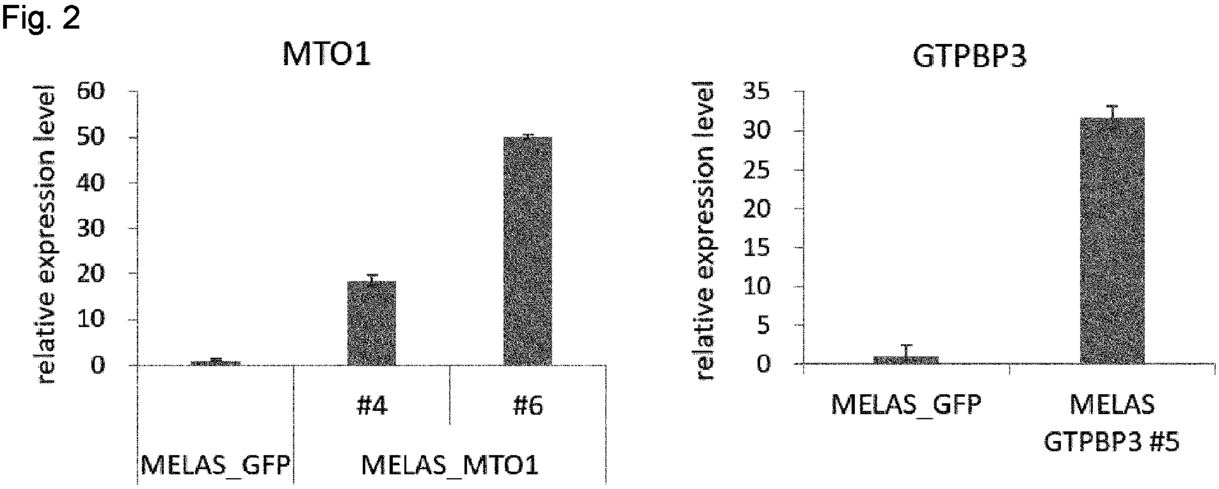
MTO1
relative expression level
60
50
40
30
20
10
0
#4
#6
MELAS_GFP
MELAS_MTO1
GTPBP3
relative expression level
35
30
25
20
15
10
5
0
MELAS_GFP
MELAS
GTPBP3 #5

Fig. 3

A

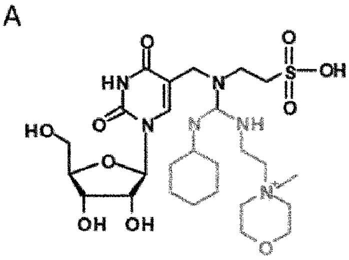

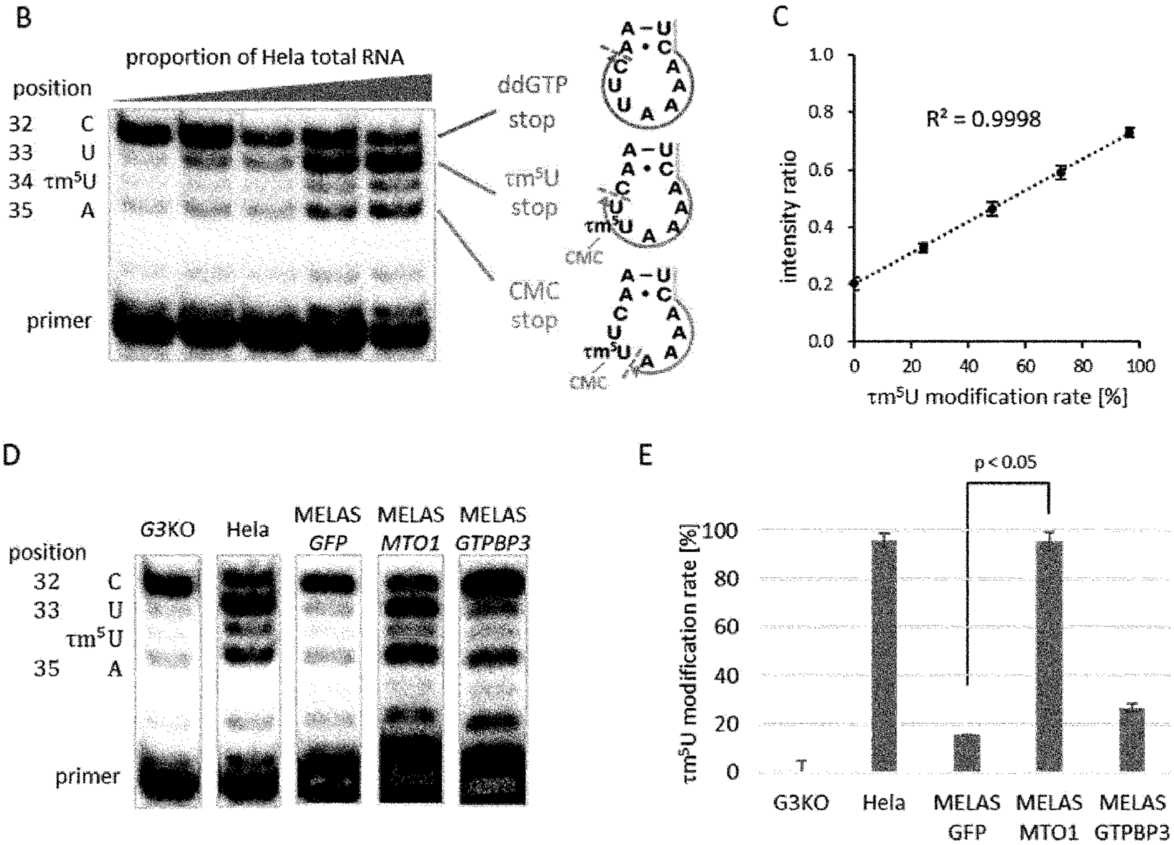
B
proportion of Hela total RNA
position
32 C
33 U
34 τm⁵U
35 A
primer
ddGTP stop
τm⁵U stop
CMC stop
CMC
C
1.0
0.8
0.6
0.4
0.2
0.0
R² = 0.9998
intensity ratio
0
20
40
60
80
100
τm⁵U modification rate [%]
D
G3KO
Hela
MELAS GFP
MELAS MTO1
MELAS GTPBP3
position
32 C
33 U
τm⁵U
35 A
primer
E
p < 0.05
τm⁵U modification rate [%]
100
80
60
40
20
0
G3KO
Hela
MELAS GFP
MELAS MTO1
MELAS GTPBP3

Fig. 4

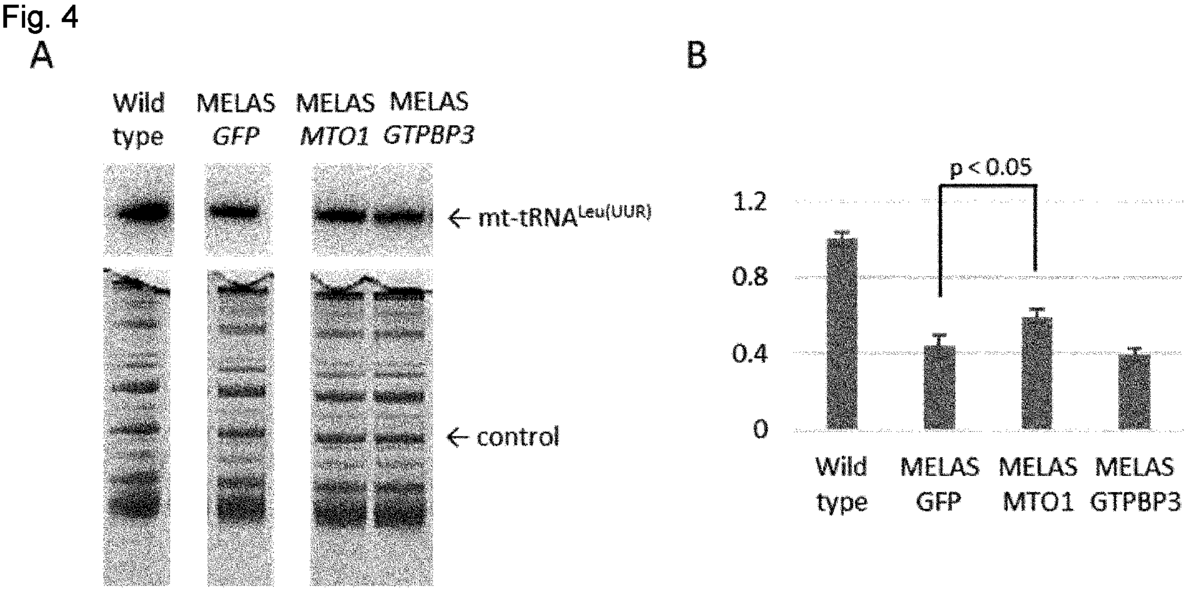
A
Wild type
MELAS GFP
MELAS MTO1
MELAS GTPBP3
← mt-tRNALeu(UUR)
← control
B
p < 0.05
1.2
0.8
0.4
0
Wild type
MELAS GFP
MELAS MTO1
MELAS GTPBP3

Fig. 5

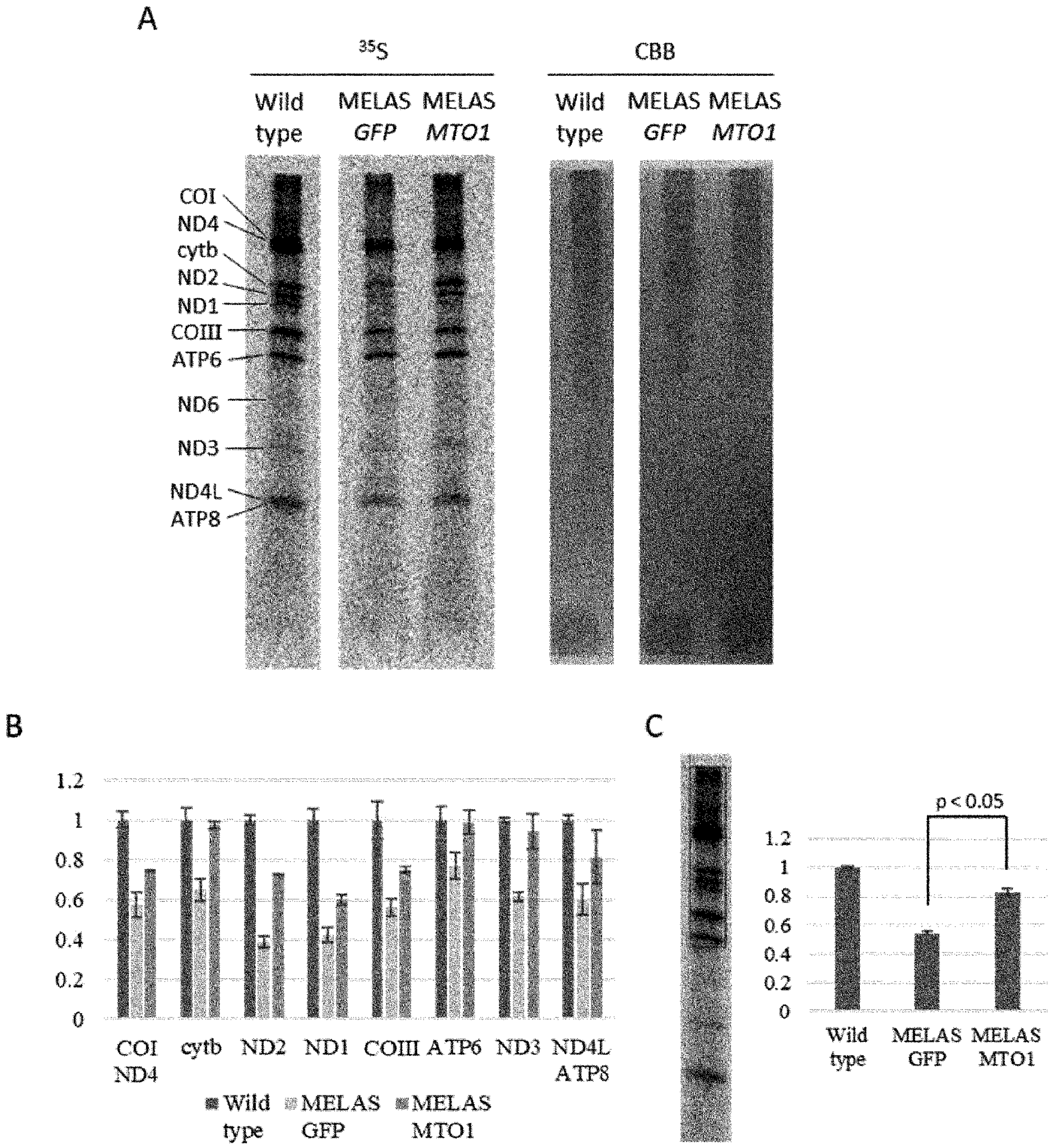
A
$^{35}$S
CBB
Wild type
MELAS GFP
MELAS MTO1
Wild type
MELAS GFP
MELAS MTO1
COI
ND4
cytb
ND2
ND1
COIII
ATP6
ND6
ND3
ND4L
ATP8
B
1.2
1
0.8
0.6
0.4
0.2
0
COI ND4
cytb
ND2
ND1
COIII
ATP6
ND3
ND4L ATP8
Wild type
MELAS GFP
MELAS MTO1
C
p < 0.05
1.2
1
0.8
0.6
0.4
0.2
0
Wild type
MELAS GFP
MELAS MTO1

Fig. 6

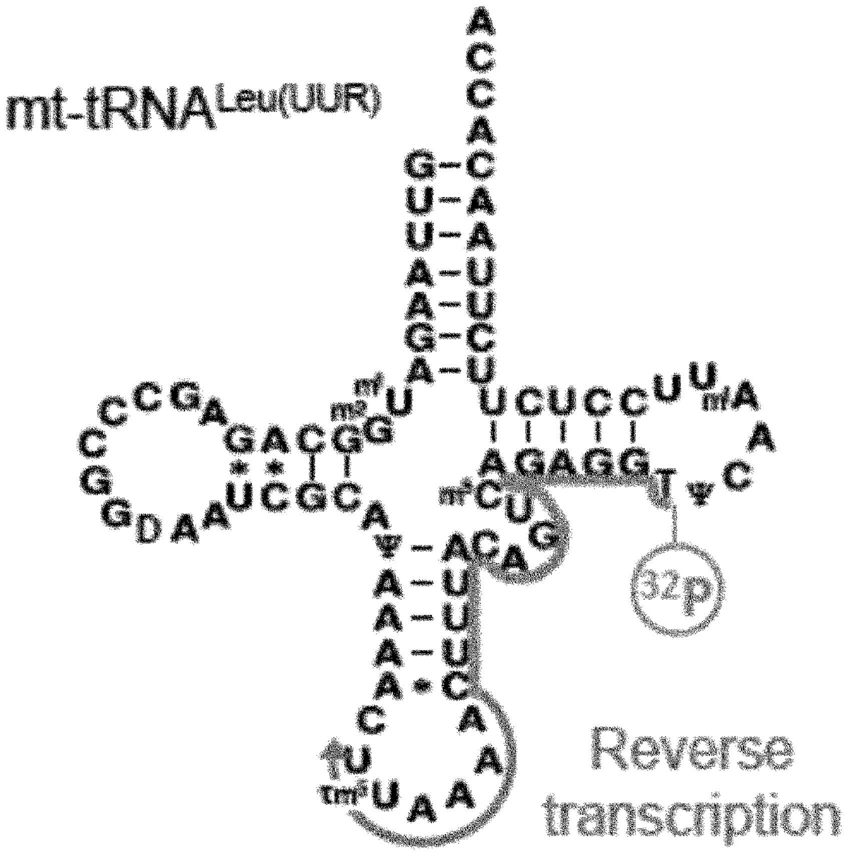
mt-tRNALeu(UUR)
32P
Reverse transcription

Fig. 7

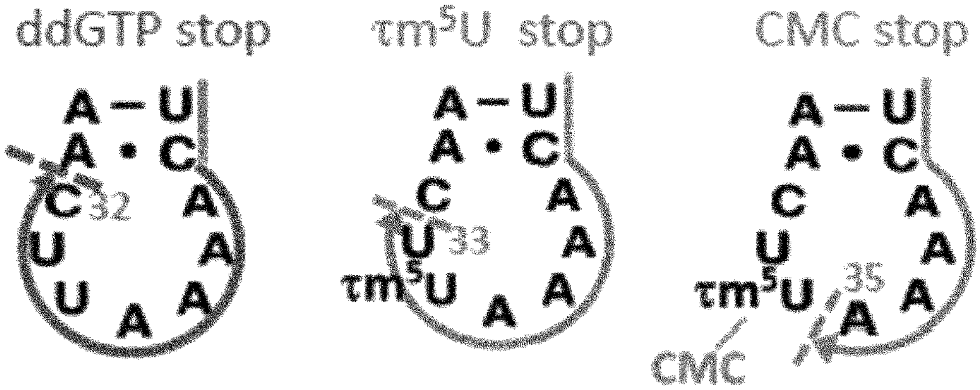
ddGTP stop
τm5U stop
CMC stop
32
33
35
CMC

Fig. 8-1

<210> 1
<211> 2079
<212> DNA
<213> Homo sapiens

<400> 1
atgttctact tccgaggctg tggccgttgg gtcgcggttt ccttcaccaa gcagcaattt 60

ccgttggcac ggttgagcag tgacagcgcg gcgccccgga ctccgcactt cgacgtgata 120

gtcattggtg gaggacatgc cgggactgag gcagccaccg ccgccgctcg gtgcggctct 180

cggactctgc tcctcactca ccgcgtggac acgatcggtc agatgtcatg taatccttcc 240

tttggtggca tcggaaaggg acatttaatg agggaagtag atgccttgga tggcctgtgt 300

tctcgcatct gtgaccagtc tggtgtacat tataaagtat taaaccggcg taagggacca 360

gctgtgtggg gtctgagagc tcagattgat aggaaactct ataaacagaa catgcagaaa 420

gaaatcttga atacaccact gcttactgtt caggagggag ctgtagaaga tcttattctt 480

acagaaccag agcctgaaca cactgggaaa tgccgtgtca gtggggttgt tttggtggat 540

ggaagcacag tatatgcaga gagtgtgatt ctgactactg ggacatttct gagaggcatg 600

attgtaattg gattggagac gcatccagca ggacgtttag gggatcagcc ttctatagga 660

ttggctcaga cactggagaa gttagggttt gtggtgggaa ggttgaagac tgggactcca 720

ccccgaattg ccaaagagtc cattaatttc agtattctaa acaagcatat accggacaat 780

ccatccatac cattcagctt taccaatgag acagtatgga ttaagccaga agatcagctg 840

ccatgttact tgactcacac caaccctaga gtggatgaga ttgtccttaa gaaccttcac 900

cttaatagtc atgttaaaga aacgacaaga ggacctcgat actgtccctc cattgaatca 960

Fig. 8-2

aaagttttgc gttttccaaa ccgtctacat caggtttggt tggaacctga aggaatggat 1020

tctgacctta tctacccaca ggggttatct atgacgctac cagctgagtt acaagagaaa 1080

atgatcacat gcatcagagg cttggagaaa gctaaagtga ttcagccagg ctacggtgtt 1140

cagtatgatt acttagatcc ccgtcagatc accccttcct tggagactca tttggttcaa 1200

cgactcttct ttgctggaca gatcaatggc accactggtt atgaggaagc tgcagctcaa 1260

ggtgtgatag ccggaatcaa cgccagtctt cgggtcagtc gcaagcctcc ctttgtggtt 1320

agccgaacag aaggttacat aggagtcttg attgatgacc tcactactct gggcaccagt 1380

gaaccatacc gcatgtttac cagccgagta gagttccgtt tgtcactgcg ccctgataat 1440

gctgacagcc ggctcacact gcgagggtat aaagacgctg gctgtgtgtc ccaacaacga 1500

tatgaaagag cttgttggat gaagtcttct ttagaagaag gcatttctgt gttgaaatct 1560

attgagtttt tgagctctaa atggaaaaaa ttaatcccag aggcttctat aagtactagt 1620

agaagtctgc ctgtcagagc tctcgatgtt ctgaagtatg aggaagttga catggattca 1680

ttagccaagg ctgttccaga gcccttgaag aagtatacta aatgtagaga gctggctgaa 1740

agactgaaaa tagaagccac ttatgaatca gtgttgttcc atcaactaca agaaataaag 1800

ggagttcagc aagatgaagc tctccaactg ccaaaagacc tagattattt gactatcagg 1860

gatgtgtctt tgtcccatga agttcgagag aaactacatt ttagtcgtcc acagacgatc 1920

ggggctgcta gtcgcatacc cggagtaaca cctgccgcca tcatcaatct gctgagattt 1980

gtgaagacca ctcaacgaag acagtcggct atgaatgaat catccaagac tgatcaatac 2040

ttatgtgatg cagacagact tcaagagaga gagttatag 2079

Fig. 9-1

<210> 2
<211> 692
<212> PRT
<213> Homo sapiens

<400> 2
Met Phe Tyr Phe Arg Gly Cys Gly Arg Trp Val Ala Val Ser Phe Thr
1 5 10 15

Lys Gln Gln Phe Pro Leu Ala Arg Leu Ser Ser Asp Ser Ala Ala Pro
20 25 30

Arg Thr Pro His Phe Asp Val Ile Val Ile Gly Gly Gly His Ala Gly
35 40 45

Thr Glu Ala Ala Thr Ala Ala Ala Arg Cys Gly Ser Arg Thr Leu Leu
50 55 60

Leu Thr His Arg Val Asp Thr Ile Gly Gln Met Ser Cys Asn Pro Ser
65 70 75 80

Phe Gly Gly Ile Gly Lys Gly His Leu Met Arg Glu Val Asp Ala Leu
85 90 95

Asp Gly Leu Cys Ser Arg Ile Cys Asp Gln Ser Gly Val His Tyr Lys
100 105 110

Val Leu Asn Arg Arg Lys Gly Pro Ala Val Trp Gly Leu Arg Ala Gln
115 120 125

Fig. 9-2

Ile Asp Arg Lys Leu Tyr Lys Gln Asn Met Gln Lys Glu Ile Leu Asn
130 135 140

Thr Pro Leu Leu Thr Val Gln Glu Gly Ala Val Glu Asp Leu Ile Leu
145 150 155 160

Thr Glu Pro Glu Pro Glu His Thr Gly Lys Cys Arg Val Ser Gly Val
165 170 175

Val Leu Val Asp Gly Ser Thr Val Tyr Ala Glu Ser Val Ile Leu Thr
180 185 190

Thr Gly Thr Phe Leu Arg Gly Met Ile Val Ile Gly Leu Glu Thr His
195 200 205

Pro Ala Gly Arg Leu Gly Asp Gln Pro Ser Ile Gly Leu Ala Gln Thr
210 215 220

Leu Glu Lys Leu Gly Phe Val Val Gly Arg Leu Lys Thr Gly Thr Pro
225 230 235 240

Pro Arg Ile Ala Lys Glu Ser Ile Asn Phe Ser Ile Leu Asn Lys His
245 250 255

Ile Pro Asp Asn Pro Ser Ile Pro Phe Ser Phe Thr Asn Glu Thr Val
260 265 270

Fig. 9-3

Trp Ile Lys Pro Glu Asp Gln Leu Pro Cys Tyr Leu Thr His Thr Asn
275 280 285

Pro Arg Val Asp Glu Ile Val Leu Lys Asn Leu His Leu Asn Ser His
290 295 300

Val Lys Glu Thr Thr Arg Gly Pro Arg Tyr Cys Pro Ser Ile Glu Ser
305 310 315 320

Lys Val Leu Arg Phe Pro Asn Arg Leu His Gln Val Trp Leu Glu Pro
325 330 335

Glu Gly Met Asp Ser Asp Leu Ile Tyr Pro Gln Gly Leu Ser Met Thr
340 345 350

Leu Pro Ala Glu Leu Gln Glu Lys Met Ile Thr Cys Ile Arg Gly Leu
355 360 365

Glu Lys Ala Lys Val Ile Gln Pro Gly Tyr Gly Val Gln Tyr Asp Tyr
370 375 380

Leu Asp Pro Arg Gln Ile Thr Pro Ser Leu Glu Thr His Leu Val Gln
385 390 395 400

Arg Leu Phe Phe Ala Gly Gln Ile Asn Gly Thr Thr Gly Tyr Glu Glu
405 410 415

Fig. 9-4

Ala Ala Ala Gln Gly Val Ile Ala Gly Ile Asn Ala Ser Leu Arg Val
420 425 430

Ser Arg Lys Pro Pro Phe Val Val Ser Arg Thr Glu Gly Tyr Ile Gly
435 440 445

Val Leu Ile Asp Asp Leu Thr Thr Leu Gly Thr Ser Glu Pro Tyr Arg
450 455 460

Met Phe Thr Ser Arg Val Glu Phe Arg Leu Ser Leu Arg Pro Asp Asn
465 470 475 480

Ala Asp Ser Arg Leu Thr Leu Arg Gly Tyr Lys Asp Ala Gly Cys Val
485 490 495

Ser Gln Gln Arg Tyr Glu Arg Ala Cys Trp Met Lys Ser Ser Leu Glu
500 505 510

Glu Gly Ile Ser Val Leu Lys Ser Ile Glu Phe Leu Ser Ser Lys Trp
515 520 525

Lys Lys Leu Ile Pro Glu Ala Ser Ile Ser Thr Ser Arg Ser Leu Pro
530 535 540

Val Arg Ala Leu Asp Val Leu Lys Tyr Glu Glu Val Asp Met Asp Ser
545 550 555 560

Fig. 9-5

Leu Ala Lys Ala Val Pro Glu Pro Leu Lys Lys Tyr Thr Lys Cys Arg
565 570 575

Glu Leu Ala Glu Arg Leu Lys Ile Glu Ala Thr Tyr Glu Ser Val Leu
580 585 590

Phe His Gln Leu Gln Glu Ile Lys Gly Val Gln Gln Asp Glu Ala Leu
595 600 605

Gln Leu Pro Lys Asp Leu Asp Tyr Leu Thr Ile Arg Asp Val Ser Leu
610 615 620

Ser His Glu Val Arg Glu Lys Leu His Phe Ser Arg Pro Gln Thr Ile
625 630 635 640

Gly Ala Ala Ser Arg Ile Pro Gly Val Thr Pro Ala Ala Ile Ile Asn
645 650 655

Leu Leu Arg Phe Val Lys Thr Thr Gln Arg Arg Gln Ser Ala Met Asn
660 665 670

Glu Ser Ser Lys Thr Asp Gln Tyr Leu Cys Asp Ala Asp Arg Leu Gln
675 680 685

Glu Arg Glu Leu
690

**INTERNATIONAL SEARCH REPORT**

International application No.
**PCT/JP2022/026887**

**A. CLASSIFICATION OF SUBJECT MATTER**

***A61K 38/43***(2006.01)i; ***A61K 31/7088***(2006.01)i; ***A61K 48/00***(2006.01)i; ***A61P 21/00***(2006.01)i; ***A61P 25/00***(2006.01)i; ***A61P 43/00***(2006.01)i; ***C07H 19/067***(2006.01)i; ***C12N 9/99***(2006.01)i; ***C12N 15/52***(2006.01)i; ***C12N 15/85***(2006.01)i; ***C12Q 1/68***(2018.01)i; ***G01N 33/15***(2006.01)i

FI: A61K38/43 ZNA; A61K31/7088; A61K48/00; A61P21/00; A61P25/00; A61P43/00 105; C07H19/067 CSP; C12N9/99; C12N15/52 Z; C12N15/85 Z; C12Q1/68; G01N33/15 Z

According to International Patent Classification (IPC) or to both national classification and IPC

**B. FIELDS SEARCHED**

Minimum documentation searched (classification system followed by classification symbols)

A61K38/43; A61K31/7088; A61K48/00; A61P21/00; A61P25/00; A61P43/00; C07H19/067; C12N9/99; C12N15/52; C12N15/85; C12Q1/68; G01N33/15

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | ASANO, K. et al. Metabolic and chemical regulation of tRNA modification associated with taurine deficiency and human disease. Nucleic Acids Research. 2018, vol. 46, pp. 1565-1583<br>p. 1569, fig. 1 | 10 |
| Y | fig. 6, 7 | 1-8 |
| A | | 9 |
| Y | BARUFFINI, E. et al. MTO1 mutations are associated with hypertrophic cardiomyopathy and lactic acidosis and cause respiratory chain deficiency in humans and yeast. Human Mutation. 2013, vol. 34, pp. 1501-1509<br>in particular, summary | 1-8 |
| A | | 9, 10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search: **22 September 2022**

Date of mailing of the international search report: **04 October 2022**

Name and mailing address of the ISA/JP
**Japan Patent Office (ISA/JP)**
**3-4-3 Kasumigaseki, Chiyoda-ku, Tokyo 100-8915**
**Japan**

Authorized officer

Telephone No.

Form PCT/ISA/210 (second sheet) (January 2015)

**INTERNATIONAL SEARCH REPORT**

International application No.
**PCT/JP2022/026887**

**C. DOCUMENTS CONSIDERED TO BE RELEVANT**

| Category* | Citation of document, with indication, where appropriate, of the relevant passages | Relevant to claim No. |
|---|---|---|
| X<br><br>A | FAKRUDDIN, M. et al. Defective mitochondrial tRNA taurine modification activates global proteostress and leads to mitochondrial disease. Cell Rep. 2018, vol. 22, pp. 482-496<br>in particular, pp. 482, 483, 494 | 1-9<br><br>10 |
| A | ZHANG, Q. et al. Ablation of Mto1 in zebrafish exhibited hypertrophic cardiomyopathy manifested by mitochondrion RNA maturation deficiency. Nucleic Acids Research. April 2021, vol. 49, no. 8, pp. 4689-4704<br>entire text | 1-10 |
| A | 森保道, ミトコンドリア糖尿病のこれまでとこれからの展望, 内分泌・糖尿病・代謝内科, 2018, vol. 47, no. 5, pp. 341-346<br>entire text, (Clinical manifestations of mitochondrial diabetes and its hopeful perspective. Endocrinology, diabetology & metabolism.), non-official translation (MORI, Yasumichi) | 1-10 |
| A | WO 2020/006125 A1 (RIBONOVA INC.) 02 January 2020 (2020-01-02) | 1-10 |
| A | WO 2020/172421 A1 (RENEO PHARMACEUTICALS, INC.) 27 August 2020 (2020-08-27) | 1-10 |

Form PCT/ISA/210 (second sheet) (January 2015)

**INTERNATIONAL SEARCH REPORT**

International application No.
**PCT/JP2022/026887**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☐ forming part of the international application as filed:
      - ☐ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

With respect to nucleotide or amino acid sequences disclosed in this international application, this international search report has been established to the extent that a meaningful international search can be conducted without a sequence table in accordance with WIPO Standard ST.26.

Form PCT/ISA/210 (continuation of first sheet) (January 2015)

**INTERNATIONAL SEARCH REPORT**

International application No.
**PCT/JP2022/026887**

**Box No. III Observations where unity of invention is lacking (Continuation of item 3 of first sheet)**

This International Searching Authority found multiple inventions in this international application, as follows:

The number of inventions in the international application set forth in claims 1-22 is 2.

(Invention 1) Claims 1-10

Claims 1-10 have the special technical feature in which mitochondrial tRNA translation optimization 1 (MTO1) or a nucleic acid encoding the same is applied in the treatment or prevention of diseases caused by reduction or deficiency of a 5-taurinomethyluridine (τm5U) modification in mitochondrial tRNA, and are thus classified as invention 1.

(Invention 2) Claims 11-22

Claims 11-22 share, with claim 1 classified as invention 1, the common technical feature of pertaining to a τm5U modification in mitochondrial tRNA. However, said technical feature does not make a contribution over the prior art in light of the disclosures of document 1 (fig. 1, etc.) and thus cannot be said to be a special technical feature. Moreover, there are no other same or corresponding special technical features between these inventions.

Further, claims 11-22 are not dependent on claim 1. Furthermore, claims 11-22 are not substantially identical to or similarly closely related to any of the claims classified as invention 1.

Accordingly, claims 11-22 cannot be classified as invention 1.

In addition, claims 11-22 have the special technical feature in which a water-soluble carbodiimide is used to detect a τm5U modification in mitochondrial tRNA, and are thus classified as invention 2.

Document 1: ASANO, K. et al. Metabolic and chemical regulation of tRNA modification associated with taurine deficiency and human disease. Nucleic Acids Research. 2018, vol. 46, pp. 1565-1583

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-10**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/JP2022/026887**

| Patent document cited in search report | Publication date (day/month/year) | Patent family member(s) | Publication date (day/month/year) |
|---|---|---|---|
| WO 2020/006125 A1 | 02 January 2020 | JP 2021-529781 A<br>US 2021/0283072 A1<br>EP 3813806 A1<br>KR 10-2021-0053877 A<br>CN 112996494 A | |
| WO 2020/172421 A1 | 27 August 2020 | JP 2022-523645 A<br>US 2022/0117972 A1<br>EP 3927718 A1<br>KR 10-2021-0134348 A<br>CN 113710683 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Nature Reviews Molecular Cell Biology,* 2021, vol. 22 (6), 375-392
- *Journal of Japanese Biochemical Society,* 2020, vol. 92 (3), 431-446
- *PNAS.,* 2005, vol. 102, 7127-7132
- **SUZUKI, T.** The expanding world of tRNA modifications and their disease relevance. *Nature Reviews Molecular Cell Biology,* 2021, vol. 22 (6), 375-392
- **SUZUKI, T.** RNA modification and biological phenomenon. *Journal of Japanese Biochemical Society,* 2020, vol. 92 (3), 431-446
- **OKAZAKI, Y. ; TOMIZAWA, K. ; SAKAGUCHI, Y. ; SUZUKI, T.** Metabolic and chemical regulation of tRNA modification associated with taurine deficiency and human disease. *Nucleic Acids Res.,* 2018, vol. 46, 1565-1583
- **OKEGAWA, Y. ; MOTOHASHI, K. A.** simple and ultra-low cost homemade seamless ligation cloning extract (SLiCE) as an alternative to a commercially available seamless DNA cloning kit. *Biochem. Biophys. Rep.,* 2015, vol. 4, 148-151
- **SASARMAN, F. ; ANTONIKA, H. ; SHOUBRIDGE, A., E.** The A3243G tRNALeu(UUR) MELAS mutation causes amino acid misincorporation and a combined respiratory chain assembly defect partially suppressed by overexpression of EFTu and EFG2. *Hum. Mol. Genet.,* 2008, vol. 17, 3697-3707
- **KIRINO, Y. ; GOTO, Y ; CAMPOS, Y ; ARENAS, J. ; SUZUKI, T.** Specific correlation between the wobble modification deficiency in mutant tRNAs and the clinical features of a human mitochondrial disease. *PNAS.,* 2005, vol. 102, 7127-7132
- **OFENGAND, J. ; DEL CAMPO, M. ; KAYA, Y.** Mapping pseudouridines in RNA molecules. *Methods,* 2001, vol. 25, 365-373
- **CHOMCZYNSKI, P. ; SACCHI, N.** Single-step method of RNA isolation by acid guanidinium thiocyanate-phenol-chloroform extraction. *Anal Biochem,* 1987, vol. 162, 156-159
- **YASUKAWA, T. ; SUZUKI, T. ; SUZUKI, T. ; UEDA, T. ; OHTA, S. ; WATANABE, K.** Modification Defect at Anticodon Wobble Nucleotide of Mitochondrial tRNAsLeu(UUR) with Pathogenic Mutations of Mitochondrial Myopathy, Encephalopathy, Lactic Acidosis, and Stroke-like Episodes. *Journal of Biological Chemistry,* 2000, vol. 275, 4251-4257